(19) Europäisches Patentamt
European Patent Office
Office européen des brevets

(11) Publication number: **0 031 221**
**B1**

(12) **EUROPEAN PATENT SPECIFICATION**

(45) Date of publication of patent specification: **02.03.83**

(21) Application number: **80304478.3**

(22) Date of filing: **11.12.80**

(51) Int. Cl.³: **A 61 K 37/02,**
C 07 C 103/52,
C 12 P 21/04 //(C12P21/04,
C12R1/045, 1/62)

(54) **Cyclic peptide nuclei.**

(30) Priority: **13.12.79 US 103121**
**13.12.79 US 103268**
**13.12.79 US 103017**
**13.12.79 US 103016**
**13.12.79 US 103313**
**25.08.80 US 181437**
**25.08.80 US 181449**
**25.08.80 US 181029**
**25.08.80 US 181443**
**25.08.80 US 181036**

(43) Date of publication of application:
**01.07.81 Bulletin 81/26**

(45) Publication of the grant of the patent:
**02.03.83 Bulletin 83/9**

(84) Designated Contracting States:
**DE GB LU NL SE**

(56) References cited:
BE - A - 883 593
DE - A - 2 704 030
DE - A - 2 742 435
US - A - 3 150 059
US - A - 3 978 210
US - A - 4 024 245
US - A - 4 024 246

(73) Proprietor: **ELI LILLY AND COMPANY**
**307, East McCarty Street**
**Indianapolis Indiana 46285 (US)**

(72) Inventor: **Abbott, Bernard John**
**3019 San Jose**
**Greenwood Indiana (US)**
Inventor: **Fukuda, David Shuichi**
**R.R.2, Box 103T**
**Brownsburg Indiana (US)**

(74) Representative: **Crowther, Terence Roger et al,**
**Erl Wood Manor**
**Windlesham Surrey GU20 6PH (GB)**

(56) References cited:
**HELVETICA CHIMICA ACTA, vol. 62, no. 129, Fasc. 4, RENE TRABER et al.: "Cyclopeptid-Antibiotika aus Aspergillus-Arten. Struktur der Echinocandine C und D" pages 1252—1266**

**TETRAHEDRON LETTERS, no 46, November 1976 C. KELLER-JUSLEN et al.: "Struktur des Cyclopeptid-Antibiotikums SL 7810 (=Echinocandin B)" pages 4147, 4148, 4150**

***Advances in Applied Microbiology*, Vol. 17 (1974) pages 315, 360**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 031 221 B1

Cyclic peptide nuclei

This invention relates to cyclic peptide nuclei of the general formula:

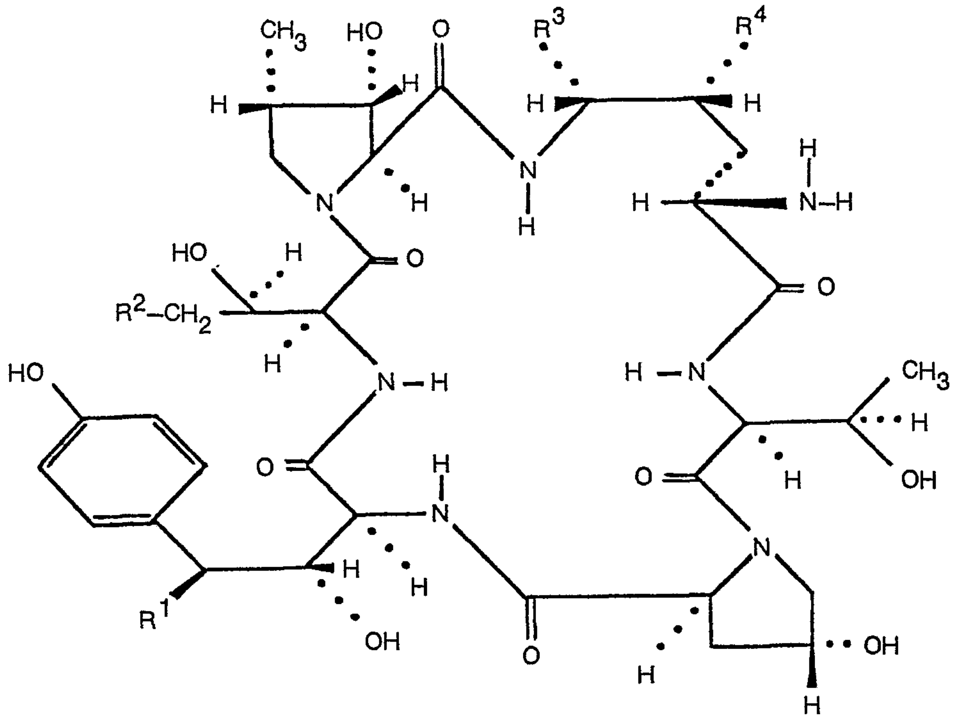

I

and acid-addition salts thereof. The nuclei and their salts are useful as intermediates in the preparation of semi-synthetic antifugal agents.

The nuclei are prepared by deacylating a cyclic peptide antibiotic of the general formula:

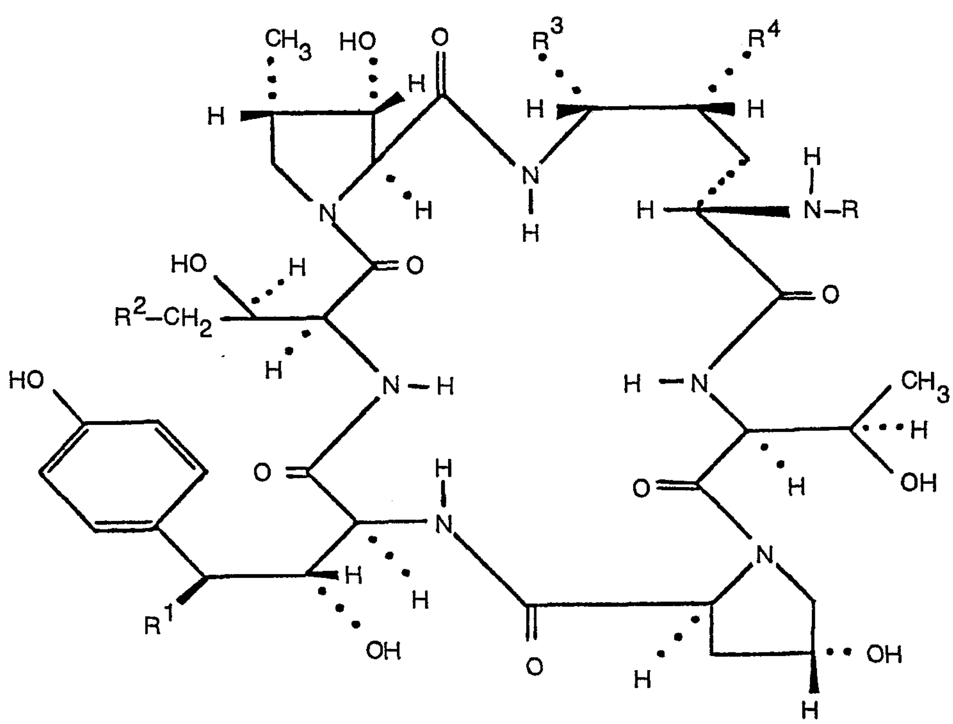

II

wherein R is a saturated or unsaturated fatty acid side chain and $R^1$, $R^2$, $R^3$ and $R^4$ are substituents defined hereinbelow.

We have discovered a process of enzymatically removing the fatty acid side chain, R, to give the cyclic peptide nucleus. The process comprises exposing the antibiotic in an aqueous medium to an enzyme produced by a microorganism of the family *Actinoplanaceae* until substantial deacylation is accomplished.

A preferred process of this invention comprises using an enzyme produced by the microorganism *Actinoplanes utahensis* NRRL 12052 to cleave the fatty acid side chain. Deacylation is ordinarily accomplished by adding the appropriate antibiotic to a culture of *A. utahensis* and permitting the culture to incubate until deacylation is accomplished. The nuclei thereby obtained are separated from the fermentation broth by methods known in the art. These nuclei are useful in that they can be reacylated to provide new antibiotic substances.

Specifically, the invention provides a cyclic peptide nucleus of the formula

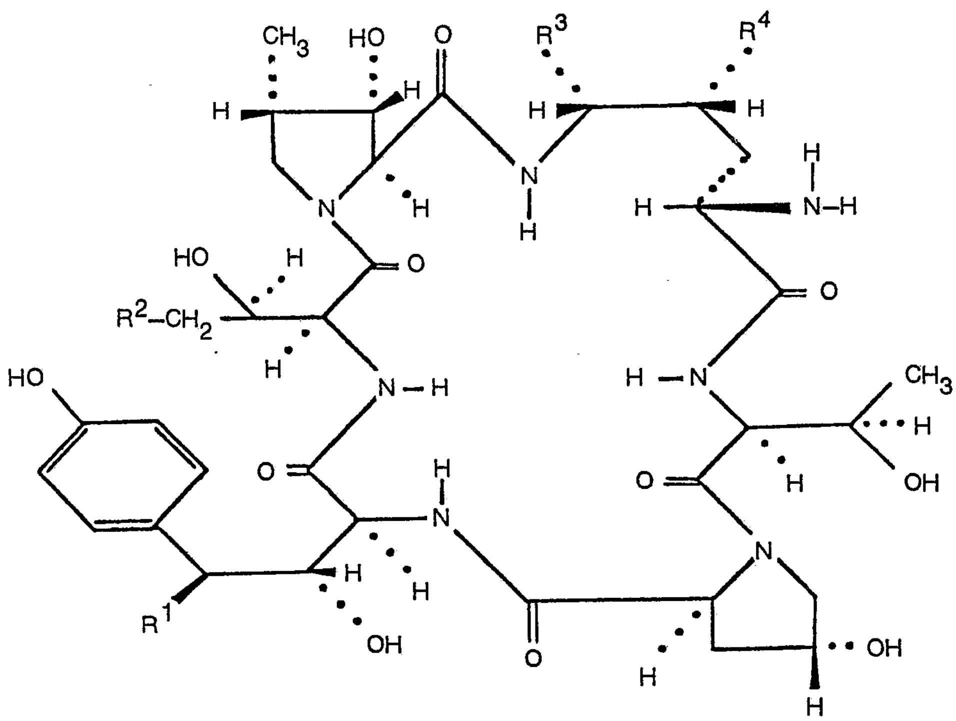

I

wherein $R^1$ is H or OH and;
when $R^1$ is H, $R^2$ is H and $R^3$ and $R^4$ are both H or both OH,
and
when $R^1$ is OH, $R^2$ is H, $R^3$ is OH or $C_1$—$C_6$ alkyloxy and $R^4$ is OH, or $R^2$ is

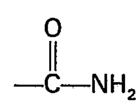

and $R^3$ and $R^4$ are both OH, and the acid addition salts thereof.

In one embodiment of the nuclei of this invention, $R^1$, $R^3$ and $R^4$ are OH and $R^2$ is H. The nucleus of this embodiment is known as A—30912A nucleus. A—30912A nucleus is prepared by deacylating a cyclic peptide antibiotic of formula II wherein R is linoleoyl, stearoyl or palmitoyl and $R^1$, $R^2$, $R^3$ and $R^4$ are as in the A-30912A nucleus.

When R in the cyclic peptide antibiotic of formula II is linoleoyl, the antibiotic is known as A—30912 factor A, when R is stearoyl the antibiotic is known as tetrahydro-A—30912 factor A, and when R is palmitoyl the antibiotic is known as aculeacin A.

A—30912 Factor A

A—30912 factor A is a factor of the A—30912 mixture which also contains factors B, C, D, E, F, and G. The A—30912 mixture and individual factors A through G are disclosed by Marvin M. Hoehn and Karl H. Michel in U.S. Patent 4,024,245. A—30912 factor A is identical to antibiotic A—22082 which is described by Calvin E. Higgens and Karl H. Michel in U.S. Patent 4,024,246.

Since the issuance of U.S. Patents 4,024,245 and 4,024,246, it has been found that A—30912 factor A is identical to the antibiotic echinocandin B [see F. Benz *et al., Helv. Chim. Acta 57,* 2459—2477 (1974) and Swiss Patent 568,386]. Antibiotic SL 7810/F has also been identified as

echinocandin B [C. Keller-Juslen, *et al., Tetrahedron Letters 1976* (46), 4147—4250, and Belgium Patent 834,289.

Keller-Juslen, *et al.*, proposed the structure of formula II wherein R=linoleoyl, $R^1$, $R^3$ and $R^4$ are OH and $R^2$ is H for antibiotic A—30912 factor A.

Tetrahydro-A—30912 Factor A

Tetrahydro-A—30912 factor A (tetrahydro-SL 7810/F; tetrahydroechinocandin B), which is described in Belgium Patent 834,289 and by F. Benz et al., *Helv. Chim Acta 57,* 2459—2477 (1974), has the structure of formula II wherein R is stearoyl, $R^1$, $R^3$ and $R^4$ are OH and $R^2$ is H. For convenience herein, this material will be called tetrahydro-A—30912A.

Aculeacin A

Aculeacin A is a component of the aculeacin mixture which consists of one major component (aculeacin A) and six minor components (aculeacins B, C, D, E, F and G). The aculeacin components are described by K. Mizuno, *et al.,* in U.S. Patent 3,978,210. As is discussed in Belgian Patent 859,067, aculeacin A probably has the same cyclic peptide structure as tetrahydro-A—30912A except that the stearoyl side chain is replaced by palmitoyl.

A—30912A Nucleus

The novel cyclic peptide nucleus of this embodiment, *i.e.,* the nucleus of A—30912 factor A (echinocandin B, SL 7810/F), tetrahydro-A—30912 factor A, and aculeacin A has the structure of formula I wherein $R^1$, $R^3$ and $R^4$ are OH and $R^2$ is H.

This nucleus (A—30912A nucleus) is a white amorphous material which is soluble in solvents such as water, dimethylformamide, dimethyl sulfoxide and methanol and which is insoluble in solvents such as chloroform, toluene, and diethyl ether.

A—30912A nucleus has an empirical formula of $C_{34}H_{51}N_7O_{15}$ and a molecular weight of 797.83.

The infrared absorption spectrum of A—30912A nucleus in KBr disc is shown in Figure I. The following absorption maxima are observed: 3340 broad (OH, H-bonded), 2970, 2930, and 2890 (CH stretch, aliphatic in $CH_3$, $CH_2$, CH groups), 1660 and 1625 (several carbonyls C=O), 1510—1550, 1430—1450 (CH wag), 1310—1340, 1230—1260, 1080, 835, 650 broad, and 550 broad $cm^{-1}$.

Electrometric titration of A—30912A nucleus in 66% aqueous dimethylformamide indicates the presence of a titratable group with a $pK_a$ value of about 7.35 (initial pH 7.32).

A—30912A nucleus can be separated by high-performance liquid chromatography (HPLC). A—30912A nucleus has an approximate retention time (k′) of 11.52 minutes when separated by HPLC using the following conditions:

Column: 4 x 300 mm

Packing: silica gel/$C_{18}$

Solvent: ammonium acetate:acetonitrile:water (1:2:97)

Flow Rate: 3 ml/min

Pressure: 2500 psi

Detector: variable wavelength UV at 230 nm

Sensitivity: 0—0.4 A.U.F.S.

Preparation of A—30912A Nucleus

Preparation of the Substrate

The A—30912A nucleus of this invention may be prepared from A—30912 factor A, tetrahydro-A—30912 factor A, or aculeacin A. These substrates may be supplied as purified materials, but it is not essential that they be purified. Thus, for example, A—30912 mixture, wherein A—30912 factor A is the major component, may be used as a substrate to prepare A—30912A nucleus.

A—30912 Factor A

A—30912 factor A may be produced by submerged aerobic fermentation of: 1) a strain of *Aspergillus rugulosus* NRRL 8113; 2) a strain of *Aspergillus nidulans* NRRL 8112; 3) a strain of *Aspergillus nidulans* var. *echinulatus* A—32204, NRRL 3860; 4) a strain of *Aspergillus rugulosus* NRRL 8039; or 5) a strain of *Aspergillus nidulans* var. *roseus,* NRRL 11440.

When a strain of *A. nidulans* var. *roseus* NRRL 11440 is used to produce A—30912 factor A, a mixture of factors is obtained which for convenience is called the A—42355 antibiotic mixture.

A—30912 factor A is the major factor of the A—42355 antibiotic mixture. A—30912 factors B, D and H are minor factors of the A—42355 mixture.

Tetrahydro-A—30912A

Tetrahydro-A—30912A is prepared from A—30912 factor A by standard hydrogenation techniques, carrying out the reduction until both double bonds of the linoleoyl side chain have been reduced.

Aculeacin A

Aculeacin A is prepared by fermentation of a strain of *Aspergillus aculeatus* NRRL 8075 as described in U.S. Patent 3,978,210 which is incorporated herein by reference.

In another embodiment of the nuclei of this invention, $R^1$ and $R^2$ are both H and $R^3$ and $R^4$ are both OH. The nucleus of this embodiment is known as A—30912B nucleus. A—30912B nucleus is prepared by deacylating a cyclic peptide antibiotic of formula II wherein R is linoleoyl or stearoyl and $R^1$, $R^2$, $R^3$ and $R^4$ are as in the A—30912B nucleus.

When R in the cyclic peptide antibiotic of formula II is linoeoyl, the antibiotic is known as A—30912 factor B and when R is stearoyl the antibiotic is known as tetrahydro-A—30912 factor B.

A—30912 Factor B

A—30912 factor B is a factor of the A—30912 mixture which also contains factors A, C, D, E, F, and G. The A—30912 mixture is described by Marvin M. Hoehn and Karl H. Michel in U.S. Patent 4,024,245.

It has been found that A—30912 factor B is identical to the antibiotic echinocandin C [see R. Traber *et al., Helv. Chim. Acta 62,* 1252—1267 (1979)] and to the antibiotic SL 7810/F—11 (Belgium Patent 834,289).

Traber, *et al.,* proposed the structure of formula II wherein $R^1$ and $R^2$ are both H, $R^3$ and $R^4$ are both OH and R is linoleoyl for the antibiotic echinocandin C. For the antibiotic, tetrahydroechinocandin C, Traber, *et al* proposed the structure of formula II wherein $R^1$ and $R^2$ are both H, $R^3$ and $R^4$ are both OH and R is stearoyl.

Tetrahydro-A—30912 Factor B

Tetrahydro-A—30912 factor B (tetrahydro-SL 7810/F—II; tetrahydroechinocandin C), which is described in Belgium Patent 834,289 and by R. Traber et al., *Helv. Chim Acta 62*, 1252—1267 (1979), has the structure of formula II wherein $R^1$ and $R^2$ are both H, $R^3$ and $R^4$ are both OH and R is stearoyl. For convenience herein, this material will be called tetrahydro-A—30912B.

## A—30912B Nucleus

The novel cyclic peptide nucleus of this embodiment, *i.e.,* the nucleus of A—30912 factor B (echinocandin C, SL 7810/F—II) and of tetrahydro-A—30912B has the structure shown in formula I, wherein $R^1$ and $R^2$ are both H and $R^3$ and $R^4$ are both OH.

A—30912B nucleus has an empirical formula of $C_{34}H_{51}N_7O_{14}$ and a molecular weight of 781.83.

## Preparation of A—30912B Nucleus

Preparation of the Substrate

The A—30912B nucleus may be prepared from A—30912 factor B or tetrahydro-A—30912B. Since A—30912 factor B is not the major component of the antibiotic mixtures wherein it is produced, it should be purified to the extent of removing the other co-produced factors before it is used as a substrate.

A—30912 Factor B

A—30912 factor B may be produced by submerged aerobic fermentation of: 1) a strain of *Aspergillus rugulosus* NRRL 8113; 2) a strain of *Aspergillus nidulans* var. *echinulatus* A 32204, NRRL 3860; 3) a strain of *Aspergillus rugulosus* NRRL 8039; or 4) a strain of *Aspergillus nidulans* var. *roseus,* NRRL 11440.

When a strain of *A. nidulans* var. *roseus* NRRL 11440 is used to produce A—30912 factor B, a mixture of factors is obtained which for convenience is called the A—42355 antibiotic mixture. A—30912 factor A is the major factor of the A—42355 antibiotic mixture. A—30912 factors B, D and H are minor factors of the A—42355 mixture.

Tetrahydro-A—30912B

Tetrahydro-A—30912B is prepared from A—30912 factor B by standard hydrogenation techniques, carrying out the reduction until both double bonds of the linoleoyl side chain have been reduced.

In a further embodiment of the nuclei of this invention, $R^1$, $R^2$, $R^3$ and $R^4$ are each H. The nucleus of this embodiment is known as A—30912D nucleus. A—30912D nucleus is prepared by deacylating a

cyclic peptide antibiotic of formula II wherein R is linoleoyl or stearoyl and $R^1$, $R^2$, $R^3$ and $R^4$ are as in the A—30912D nucleus.

When R in the cyclic peptide antibiotic of formula II is linoleoyl, the antibiotic is known as A—30912 factor D and when R is stearoyl the antibiotic is known as tetrahydro-A—30912 factor D.

A—30912 Factor D

A—30912 factor D is a factor of the A—30912 mixture which also contains factors A, B, C, E, F, and G. The A—30912 mixture is described by Marvin M. Hoehn and Karl H. Michel in U.S. Patent 4,024,245.

It has been found that A—30912 factor D is identical to the antibiotic echinocandin D [see R. Traber *et al., Helv. Chim. Acta 62,* 1252—1267 (1979)] and to the antibiotic SL 7810/F—III (Belgium Patent 834,289).

Traber, *et al.,* proposed the structure of formula II wherein $R^1$, $R^2$, $R^3$ and $R^4$ are each H and R is linoleoyl for the antibiotic echinocandin D. For the antibiotic tetrahydrochinocandin D, Traber, *et al.,* proposed the structure of formula II wherein $R^1$, $R^2$, $R^3$ and $R^4$ are each H and R is stearoyl.

For convenience herein, tetrahydro-A—30912 factor D (tetrahydro-SL 7810/F—III; tetrahydroechinocandin D) will be called tetrahydro-A—30912D.

A—30912D Nucleus

The novel cyclic peptide nucleus of this embodiment, *i.e.,* the nucleus of A—30912 factor D (echinocandin D, SL 7810/F—III) and of tetrahydro-A—30912D has the structure shown in formula I, wherein $R^1$, $R^2$, $R^3$ and $R^4$ are each H.

A—30912D nucleus has an empirical formula of $C_{34}H_{51}N_7O_{12}$ and a molecular weight of 749.83.

Preparation of A—30912D Nucleus

Preparation of the Substrate

The A—30912D nucleus may be prepared from A—30912 factor D or tetrahydro-A—30912D. Since A—30912 factor D is not the major component of the antibiotic mixtures in which it is produced, it should be purified to the extent of removing the other co-produced factors before it is used as a substrate.

A—30912 Factor D

A—30912 factor D may be produced by submerged aerobic fermentation of: 1) a strain of *Aspergillus rugulosus* NRRL 8113; 2) a strain of *Aspergillus nidulans* var. *echinulatus* A 32204, NRRL 3860; 3) a strain of *Aspergillus rugulosus* NRRL 8039; or 4) a strain of *Aspergillus nidulans* var. *roseus,* NRRL 11440.

When a strain of *A. nidulans* var. *roseus* NRRL 11440 is used to produce A—30912 factor D, a mixture of factors is obtained which for convenience is called the A—42355 antibiotic mixture. A—30912 factor A is the major factor of the A—42355 antibiotic mixture. A—30912 factors B, D and H are minor factors of the A—42355 mixture.

Tetrahydro-A—30912D

Tetrahydro-A—30912D is prepared from A—30912 factor D by standard hydrogenation techniques, carrying out the reduction until both double bonds of the linoleoyl side chain have been reduced.

In yet another embodiment of the nuclei of this invention, $R^1$ and $R^4$ are both OH, $R^2$ is H and $R^3$ is $C_1$—$C_6$ alkyloxy. The nuclei of this embodiment are known as A—30912H-type nuclei. The A—30912H-type nuclei are prepared by deacylating a cyclic peptide antibiotic of formula II wherein R is linoleoyl or stearoyl and $R^1$, $R^2$, $R^3$ and $R^4$ are as in the A—30912H-type nuclei.

When R in the cyclic peptide antibiotic of formula II is linoleoyl and $R^3$ is methoxy the antibiotic is known as A—30912 factor H and when R is stearoyl and $R^3$ is methoxy, the antibiotic is known as tetrahydro-A—30912 factor H.

When R in the cyclic peptide antibiotic of formula II is linoleoyl and $R^3$ is $C_2$—$C_6$ alkyloxy, the antibiotics are known as the lower alkyloxy homologs of A—30912 factor H and when R is stearoyl and $R^3$ is $C_2$—$C_6$ alkyloxy, the antibiotics are known as the lower alkyloxy homologs of tetrahydro-A—30912 factor H.

A—30912 Factor H

A—30912 factor H is a factor of the A—30912 mixture which also contains factors A, C, D, E, F and G. The A—30912 mixture is described by Marvin, M. Hoehn and Karl H. Michel in U.S. Patent 4,024,245. A—30912 factor H was a later-discovered A—30912 factor. A—30912 factor H is discussed in a co-pending application No. 80301913.2 published as EP—A—21685 and entitled "A—30912H-type antibiotics their preparation and use".

Homologs of A—30912 Factor H

Following the discovery of the structure of A—30912 factor H, the fact that lower alkyloxy homologs of A—30912 factor H would be useful products became appreciated. Prior to this time, the alkyloxy derivatives which had been prepared were not recognized as having a useful purpose and were prepared only as structure determination tools. The lower $C_2$—$C_6$ alkyloxy homologs of A—30912 factor H are prepared from A—30912 factor A.

A—30912 factor A may be produced by fermentation of: 1) a strain of *Aspergillus rugulosus* NRRL 8113; 2) a strain of *Aspergillus nidulans* NRRL 8112; 3) a strain of *Aspergillus nidulans* var. *echinulatus* A—32204, NRRL 3860: 4) a strain of *Aspergillus rugulosus* NRRL 8039 as described in Belgian Patent 834,289; or 5) by a strain of *Aspergillus nidulans* var. *roseus*, NRRL 11440.

Because each A—30912H nuclei contains an amino moiety, it may exist in the form of salts. Such salts are also useful as intermediates and for purification purposes. The pharmaceutically acceptable salts of the A—30912H nuclei are especially useful because purification of final products will be minimized. "Pharmaceutically acceptable" salts refer to those salts in which the toxicity of product as a whole toward warm-blooded animals is not increased.

Acid addition salts of A—30912H nuclei may be formed by standard reaction procedures with an inorganic or organic acid. Representative inorganic and organic acids include hydrochloric, hydrobromic, hydriodic, sulfuric, phosphoric, acetic, benzoic, sulfamic, tartaric, citric, maleic, succinic, ascorbic, glycolic, lactic, fumaric, palmitic, cholic, pamoic, mucic, D-glutamic, *d*-camphoric, glutaric, phthalic, lauric, stearic, salicylic, methanesulfonic, benzenesulfonic, sorbic, picric, cinnamic, and other suitable acids.

## Preparation of A—30912H Nuclei

Preparation of the Substrate

The A—30912H nucleus, i.e. the compound of formula I wherein $R^1$ and $R^4$ are both OH, $R^2$ is H and $R^3$ is methoxy, can be prepared from A—30912 factor H or tetrahydro-A—30912H. Since A—30912 factor H is not a major component of the antibiotic mixtures in which it is produced, it should be purified to the extent of removing the other co-produced antibiotic factors before its use as a substrate.

The substrates for the preparation of the A—30912H nuclei of formula I wherein $R^1$ and $R^4$ are both OH, $R^2$ is H and $R^3$ is $C_2$—$C_6$ alkyloxy (the A—30912H homologs) are prepared by reaction of A—30912 factor A or tetrahydro-A—30912A with an appropriate alcohol to prepare the corresponding $C_2$—$C_6$ alkyloxy derivative. Since A—30912 factor A is the major component of the antibiotic mixtures in which it is produced, this process is also a preferred way of preparing A—30912 factor H and tetrahydro-A—30912H.

A—30912 Factor H

A—30912 factor H may be produced by submerged aerobic fermentation of a strain of *Aspergillus rugulosus* NRRL 8113 or by a strain of *Aspergillus nidulans* var. *roseus* NRRL 11440.

When a strain of *A. nidulans* var. *roseus* NRRL 11440 is used to produce A—30912 factor H, a mixture of factors is obtained which for convenience is called the A—42355 antibiotic mixture. A—30912 factor A is the major factor of the A—42355 antibiotic mixture. A—30912 factors B, D and H are minor factors of the A—42355 mixture.

The A—30912H Homologs

The A—30912H homologs are prepared by reacting A—30912 factor A or tetrahydro-A—30912A with the appropriate corresponding alcohol to form the desired alkyloxy derivative of the structure of formula II wherein $R^1$ and $R^4$ are both OH, $R^2$ is H and $R^3$ is $C_2$—$C_6$ alkyloxy. This is a preferred process of preparing A—30912 factor H and tetrahydro-A—30912H. The A—30912H homologs of formula II wherein R is stearoyl and $R^3$ is $C_2$—$C_6$ alkyloxy can be prepared by a) preparing tetrahydro-A—30912A and reacting with the appropriate alcohol to form the alkyloxy derivative, or b) reacting A—30912 factor A with the appropriate alcohol to form the alkyloxy derivative and then reducing the double bonds of the linoleoyl side chain.

The Tetrahydro Derivatives

Tetrahydro-A—30912A, tetrahydro-A—30912H, and the compounds of formula II wherein $R^3$ is $C_2$—$C_6$ alkyloxy and R is stearoyl are prepared from A—30912 factors A and H and from the compounds of formula II wherein $R^3$ is $C_2$—$C_6$ alkyloxy and R is linoleoyl by standard hydrogenation techniques, carrying out the reduction until both double bonds of the linoleoyl side chain have been reduced.

In a still further embodiment of the nuclei of this invention, $R^1$, $R^3$ and $R^4$ are OH and $R^2$ is carboxamide. The nucleus of this embodiment is known as S 31794/F—1 nucleus. S 31794/F—1 nucleus is prepared by deacylating a cyclic peptide antibiotic of formula II wherein R is myristoyl and $R^1$, $R^2$, $R^3$ and $R^4$ are as in the S 31794/F—1 nucleus.

S 31794/F—1

The S 31794/F—1 nucleus of this invention is obtained by deacylating the cyclic peptide antibiotic S 31794/F—1.

Antibiotic S 31794/F—1, which is disclosed in German Offenlegungschrift 2,628,965 and in U.S. Patent 4,173,629, is an antifungal compound produced by *Acrophialophora limonispora* nov. spec. Dreyfuss et Muller NRRL 8095. S 31794/F—1 has the following characteristics: m.p. 178—180°C (dec.) (amorphous) or 181—183°C. (dec.) (crystalline); $[\alpha]_D^{20}$ −24° (*c* 0.5, $CH_3OH$) or +37° (*c* 0,5, methanol (crystalline); UV absorption maxima in methanol at 194 nm ($E_{1cm}^{1\%}$ = 807), 225 nm (shoulder) $E_{1cm}^{1\%}$ = 132), 276 nm ($E_{1cm}^{1\%}$ = 12.8) 284 nm (shoulder) ($E_{1cm}^{1\%}$ = 10.5); $^{13}C$—NMR spectrum in deuteromethanol (190 mg in 1.5 ml deuteromethanol, tetramethylsilane as internal standard) with the following characteristics (crystalline):

| PPM | PPM | PPM |
|---|---|---|
| 176.2 | 75.5 | 51.2 |
| 175.0 | 74.0 | 39.7 |
| 173.7 | 71.0 | 38.8 |
| 172.6 | 70.5 | 36.6 |
| 172.0 | 69.7 | 34.8 |
| 171.8 | 68.0 | 32.8 |
| 171.7 | 62.2 | 30.6 |
| 168.6 | 58.3 | 26.7 |
| 157.7 | 57.0 | 23.5 |
| 132.5 | 56.2 | 19.7 |
| 129.0 | 55.4 | 14.3 |
| 115.9 | 52.9 | 11.1 |
| 76.6 | | |

an approximate elemental analysis (after drying crystalline material for two hours in a high vacuum at 100°C) as follows: 55.5—56.5 percent carbon, 7.5—7.7 percent hydrogen, 10.5—10.8 percent nitrogen and 25.5—26.0 percent oxygen. S 31794/F—1 is readily soluble in methanol, ethanol, pyridine, dimethyl sulfoxide and poorly soluble in water, chloroform, ethyl acetate, diethyl ether, benzene and hexane; and has antifungal activity, especially against *Candida albicans.*

Antibiotic S 31794/F—1 is believed to have the structure of formula II wherein R = myristoyl, $R^1$, $R^3$ and $R^4$ are OH and $R^2$ is carboxamide.

### S 31794/F—1 Nucleus

The novel cyclic peptide nucleus of this invention, *i.e.,* the nucleus of antibiotic S 31794/F—1 is believed to have the structure shown in formula I, wherein $R^1$, $R^3$ and $R^4$ are OH and $R^2$ is carboxamide.

S 31794/F—1 nucleus has an empirical formula of $C_{35}H_{52}N_8O_{16}$ and a molecular weight of 840.87.

### Preparation of S 31794/F—1 Nucleus

Preparation of the Substrate

The S 31794/F—1 nucleus of this invention is prepared from antibiotic S 31794/F—1.

Antibiotic S 31794/F—1 is prepared by submerged aerobic cultivation of *Acrophialophora limonispora* NRRL 8095. This microorganism is a part of the permanent culture collection of the Northern Regional Research Center, U.S. Department of Agriculture, Agricultural Research Culture Collection, North Central Region, Peoria, Illinois 61604, from which it is available to the public under the designated NRRL number.

Because the nuclei each contain an amino moiety, they may exist in the form of salts. Such salts are also useful as intermediates and for purification purposes. The pharmaceutically acceptable salts of

the nuclei are especially useful because purification of final products will be minimized. "Pharmaceutically acceptable" salts refer to those salts in which the toxicity of product as a whole toward warm-blooded animals is not increased.

Acid addition salts of the nuclei may be formed by standard reaction procedures with an inorganic or organic acid. Representative inorganic and organic acids include hydrochloric, hydrobromic, hydriodic, sulfuric, phosphoric acetic, benzoic, sulfamic, tartaric, citric, maleic, succinic, ascorbic, glycolic, lactic, fumaric, palmitic, cholic, pamoic, mucic, D-glutamic, *d*-camphoric, glutaric, phthalic, lauric, stearic, salicylic, methanesulfonic, benzenesulfonic, sorbic, picric, cinnamic, and other suitable acids.

Reversed-phase high performance, low pressure liquid chromatography (HPLPLC) using silica gel/$C_{18}$ adsorbent is a preferred process for the final purification of the individual antibiotics. In this process the crude antibiotic mixture, dissolved in solvent, is placed on a column equilibrated with the same solvent. The column is then eluted with solvent. Fractions collected are monitored by *Candida albicans* bioautography and/or by UV (based on relative retention times). Fractions containing identical individual antibiotic factors are combined. It is sometimes necessary to carry out an additional chromatographic separation in order to obtain the individual factors in purified form.

The crude antibiotic mixture of A—30912 or A—42355 is obtained, for example, by extraction of the whole broth or mycelia with methanol and chloroform. Methanol:water:acetonitrile (7:2:1) is a preferred solvent system for the chromatography of these antibiotics.

The crude S 31794/F—1 antibiotic is obtained by extracting the whole broth with ethyl acetate: isopropanol (4;1) and chromatographing the extract.

The individual factors can be identified by the use of thin-layer chromatography (TLC). Silica gel is a preferred adsorbent.

The $R_f$ values of A-30912 factors A—G, using high carbon content silica gel TLC, a benzene: methanol (7:3) solvent system, and *Candida albicans* bioautography are given in Table I.

TABLE I

| A—30912 Factor | $R_f$ Value |
|---|---|
| A | 0.35 |
| B | 0.45 |
| C | 0.54 |
| D | 0.59 |
| E | 0.27 |
| F | 0.18 |
| G | 0.13 |

The approximate $R_f$ values of A—30912 factors A, B, C, D, and H in different solvent systems, using high carbon content silica gel TLC and *Candida albicans* bioautography, are given in Table II.

TABLE II

| A—30912 Factor | $R_f$ Values—Solvent Systems | | | |
|---|---|---|---|---|
| | *a* | *b* | *c* | *d* |
| Factor A | 0.28 | 0.14 | 0.28 | 0.43 |
| Factor B | 0.39 | 0.21 | 0.42 | 0.47 |
| Factor C | 0.46 | 0.31 | 0.51 | 0.58 |
| Factor D | 0.50 | 0.38 | 0.57 | 0.61 |
| Factor H | 0.42 | 0.27 | 0.36 | 0.53 |

Solvent Systems

*a*: ethyl acetate:methanol (3:2)

*b*: ethyl acetate:methanol (7:3)

*c*: acetonitrile:water (95:5)

*d*: ethyl acetate:ethanol:acetic acid (40:60:0.25)

A—30912 factors A, B, D and H can also be identified by analytical HPLPLC using the following conditions:

| | |
|---|---|
| Column: | glass, 0.8 × 15.0 cm |
| Packing: | Nucleosil® 10-$C_{18}$ (Machery-Nagel and Company); packed using slurry-packing procedure of Example 7 |
| Solvent: | methanol:water:acetonitrile (7:2:1) |
| Sample Volume: | 8 mcl |
| Sample Size: | 8 mcg |
| Column Temperature: | ambient |
| Flow Rate: | 1.8 ml/min |
| Pressure: | *ca.* 200 psi |
| Detector: | UV at 222 nm (ISCO Model 1800 Variable Wavelength UV-Visible Absorbance Monitor) |
| Pump: | LDC Duplex Minipump |
| Injection: | loop injection |

The approximate retention times for A—30912 factors A, B, D, and H under these conditions are summarized in Table III.

Table III

| A—30912 Factor | Retention Time (seconds) |
|---|---|
| A | 792 |
| B | 870 |
| H | 990 |
| D | 1,140 |

Preparation of the Enzyme

1. The Producing Microorganism

The enzyme which is useful for deacylation of the antibiotics of this invention is produced by certain microorganisms of the family *Actinoplanaceae,* preferably the microorganism *Actinoplanes utahensis* NRRL 12052.

The enzyme may be the same enzyme which has been used to deacylate penicillins; this work is described by Walter J. Kleinschmidt, Walter E. Wright, Frederick W. Kavanagh, and William M. Stark in U.S. Patent 3,150,059. Although a preferred method of cultivating *A. utahensis* NRRL 12052 to produce this enzyme is described in Preparation 1, it will be recognized by those skilled in the art that other methods may be used.

The *Actinoplanaceae* are a comparatively recent family of microorganisms of the order Actinomycetales. First described by Dr. John N. Couch, this family was established in 1955 [*J. Elisha Mitchell Sci. Soc. 71,* 148—155 (1955)]. The characteristics of the family and of many individual genera are found in "Bergey's Manual of Determinative Bacteriology", 8th ed., R. E. Buchanan and N. E. Gibbons, Eds., The Williams & Wilkins Co., Baltimore, Md., 1974, pages 706—723. Ten genera have thus far been distinguished: I. *Actinoplanes* (the type genus and thus far the most common genus); II. *Spirillospora;* III. *Streptosporangium;* IV. *Amorphosporangium;* V. *Ampullariella;* VI. *Pilimelia;* VII. *Planomonospora;* VIII. *Planobispora;* IX. *Dactylosporangium;* and X. *Kitasatoa.*

Some of the species and varieties which have been isolated and characterized so far are: *Actinoplanes philippinensis, Actinoplanes armeniacus, Actinoplanes utahensis,* and *Actinoplanes missouriensis; Spirillopspora albida; Streptosporiangium roseum, Streptosporangium vulgare, Streptosporangium roseum* var. *hollandensis, Streptosporangium album, Streptosporangium viridialbum, Amorphosporangium auranticolor, Ampullariella regularis, Ampullariella campanulata, Ampullariella lobata, Ampullariella digitata, Pilimelia terevasa, Pilimelia anulata, Planomonospora parontospora, Planomonospora venezuelensis, Planobispora longispora, Planobispora rosea, Dactylosporangium aurantiacum,* and *Dactylosporangium thailandense.*

The genus *Actinoplanes* is a preferred source of the enzyme which is useful for this invention. Within the genus *Actinoplanes,* the species *Actinoplanes utahensis* is an especially preferred source of the enzyme.

Cultures of representative species are available to the public from the Northern Regional Research Center, address *supra,* under the following accession numbers:

| | | |
|---|---|---|
| *Actinoplanes utahensis* | NRRL | 12052 |
| *Actinoplanes missouriensis* | NRRL | 12053 |
| *Actinoplanes sp.* | NRRL | 8122 |
| *Actinoplanes sp.* | NRRL | 12065 |
| *Streptosporangium roseum* var. *hollandensis* | NRRL | 12064 |

*A. utahensis* NRRL 12052 was derived from a parent culture which was deposited with the American Type Culture Collection (ATCC), 12301 Parklawn Drive, Rockville, Md. 20852 (*A. utahensis* ATCC 14539). The *A. utahensis* ATCC 14539 culture may also be used as a source of the enzyme.

*A. missouriensis* NRRL 12053 was derived from a culture which was also deposited with ATCC (*A. missouriensis* ATCC 14538) and which is another source of the enzyme.

The effectiveness of any given strain of microorganism within the family *Actinoplanaceae* for carrying out the deacylation of this invention is determined by the following procedure. A suitable growth medium is inoculated with the microorganism. The culture is incubated at about 28°C. for two or three days on a rotary shaker. One of the substrate antibiotics is then added to the culture. The pH of the fermentation medium is maintained at about pH 6.5. The culture is monitored for activity using a *Candida albicans* assay. This procedure is described in Sect. E. Loss of antibiotic activity is an indication that the microorganism produces the requisite enzyme for deacylation. This must be verified, however, using one of the following methods: 1) analysis by HPLC for presence of the intact nucleus; or 2) reacylation with an appropriate side chain to restore activity.

2. Conditions for Enzyme Production

Production of the enzyme occurs under conditions satisfactory for growth of the *Actinoplanaceae,* i.e., a temperature between about 25 and about 30°C. and a pH of between about 5.0 and about 8.0, with agitation and aeration. The culture medium should contain a) an assimilable carbon source such as sucrose, glucose, glycerol, or the like; b) a nitrogen source such as peptone, urea, ammonium sulfate, or the like; c) a phosphate source such as a soluble phosphate salt; and d) inorganic salts found generally to be effective in promoting the growth of microorganisms. An effective amount of the enzyme is generally obtained in from about 40 to about 60 hours after the beginning of the growth cycle and persists for some time after the effective growth has been reached. The amount of enzyme produced varies from species to species of the organism and in response to different growth conditions.

As will be apparent to those in the field, the microorganisms, such as *Actinoplanes utahensis* NRRL 12052, which produce the enzyme are subject to variation. For example, artificial variants and mutants of these strains may be obtained by treatment with various known mutagens such as

ultraviolet rays, X-rays, high-frequency waves, radioactive rays, and chemicals. All natural and artificial variants and mutants which are obtained from the *Actinoplanaceae* and which produce the enzyme may be used in this invention.

C. Deacylation Conditions

The substrate is preferably added to the culture of *Actinoplanaceae* after the culture has been incubated for at least about 48 hours. The concentration of substrate in the conversion medium can vary widely. For maximum use of the enzyme and for substantially complete deacylation within a 24-hour period, however, the concentration of substrate will generally range from about 0.5 to about 1.0 mg/ml. Lower concentrations can be used, but may not make maximum use of the enzyme; higher concentrations can also be used, but the substrate may not be completely deacylated unless the fermentation time is extended.

Conversion of the substrate antibiotics to the corresponding nuclei of this invention proceeds best when the pH of the fermentation medium is maintained in the range of from about 6.0 to about 7.0. At pH 6 or below, deacylation proceeds slowly; as pH values move above pH 6.0, both the substrate and the nucleus which is formed are increasingly less stable. For maximum stability, a pH of 6.0 is preferred; but at pH 6.0 the deacylation will occur less rapidly (about 30 to 36 hours). For more rapid deacylation (about 24 hours) without major losses, a pH of about 6.5 is preferred. In stirred fermentors the pH may be controlled by sensor controllers. Where this is impractical, such as in flask fermentors, pH may be controlled by adding 0.1 molar phosphate buffer to the medium prior to addition of the substrate.

After addition of the substrate, incubation of the culture should be continued for about 24 hours or longer. The purity of the substrate will affect the rate of deacylation. For example, substrate having a purity of greater than 50 percent is deacylated at a rate of about 0.8 to 1.2 mg/ml of antibiotic in 24 hours. When substrates of lower purity are used, the deacylation proceeds at a slower rate.

Multiple substrate feedings may be made. For example, in small tanks 0.3—0.5 mg/ml of antibiotic may be fed at 12-hour intervals for at least five additions, and in larger tanks 0.7 mg/ml may be fed twice.

The deacylation can be carried out over a broad temperature range, e.g. from about 20 to about 45°C. It is preferable, however, to carry out the deacylation at temperatures of from about 25 to about 30°C., especially preferred is a temperature of about 26°C., for optimum deacylation and stability of substrate and nucleus.

D. The Substrate

It is preferable to use purified antibiotics as the substrates. The substrate antibiotics have antifungal, but no antibacterial, activity. Thus, the substrate materials may harbour bacterial cells or spores which could grow in the deacylation fermentation medium. Such contaminants can affect the deacylation reaction or the stability of the starting antibiotics or the product nuclei. It is important, therefore, that the substrates be sterile. Since autoclaving destroys most of the substrate antibiotics, it is preferable to sterilize preparations with ethylene oxide treatment in a pressurized system.

E. Monitoring the Deacylation

The starting materials are antifungal antibiotics which are especially active against *Candida albicans.* For this reason an assay using *C. albicans* is preferable for determining quantities of substrates present. The nuclei which are formed are water soluble, but are biologically inactive. Reduction in biological activity is, therefore, a quick, presumptive test for deacylation. Both broth samples and alcoholic extracts of the fermentation solids should be assayed because the substrates are only slightly soluble in the broth.

F. Use of Resting Cells

An alternate process of deacylation involves removing the *Actinoplanaceae* cells from the culture medium, resuspending the cells in a buffer solution, and carrying out the deacylation as described in Sect. C. When this process is used, the enzymatically active mycelia can be re-used. For example, *A. utahensis* NRRL 12052 mycelia retain deacylase activity after storage for one month or longer under refrigeration (4—8°C.) or in the frozen state (—20°C.). A preferred buffer solution is 0.1 molar phosphate buffer.

G. Immobilized Enzymes

Yet another process of carrying out the deacylation is to immobilize the enzyme by processes known in the art. (See, for example, "Biomedical Applications of Immobilized Enzymes and Proteins", Thomas Ming Swi Chang, Ed., Plenum Press, New York, 1977; Vol. 1.) The immobilized enzyme can then be used in a column (or other suitable type of reactor) to effect the deacylation.

In addition, the microorganism itself can be immobilized and used to catalyze the deacylation reaction.

Utility of the Nuclei

The nuclei and their acid-addition salts are useful intermediates in the preparation of synthetic antifungal compounds. Useful antifungal compounds of formula III prepared from these nuclei are described in a copendng application of Bernard J. Abbott and David S. Fukuda, No. 80304477.5 published as EP—A—31662, and in two co-pending applications of Manuel Debono, No. 80304471.8, published as EP—A—32009 and No. 80304470.0 published as EP—A—31220, all of which are entitled DERIVATIVES OF CYCLIC PEPTIDE NUCLEI and which are filed herewith this even date.

Subject to the aforementioned provisos, the term "alkyl" means a univalent, saturated, straight-chain or branched-chain hydrocarbon radical. The term "alkenyl" means a univalent, unsaturated, straight-chain or branched-chain hydrocarbon radical containing not more than three double bonds. The double bonds of the unsaturated hydrocarbon chain may be either in the *cis* or *trans* configuration. By "$C_6$—$C_{24}$" is meant a hydrocarbon (including straight and branched chains) containing from 6 to 24 carbon atoms.

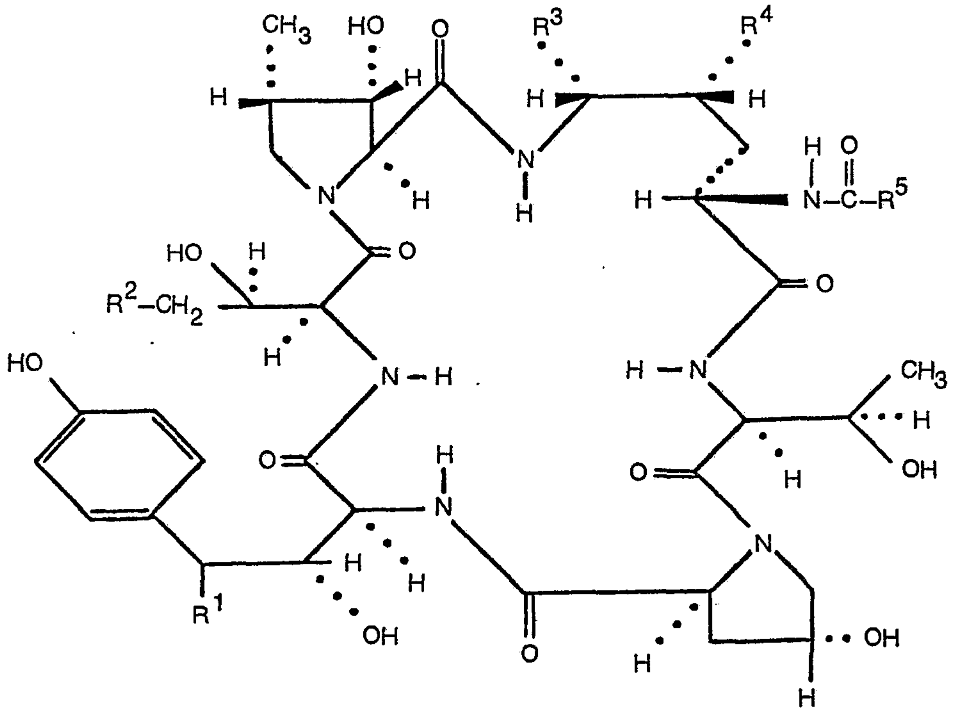

III

The compounds of formula III are prepared by acylating the appropriate nucleus at the $\alpha$-amino group of the ornithine portion of the nucleus with the appropriate acyl side chain using methods conventional in the art for forming an amide bond. The acylation is accomplished, in general, by reacting the nucleus with an activated derivative of the acid corresponding to the desired acyl side chain group.

The term "activated derivative" means a derivative which renders the carboxyl function of the acylating agent reactive to coupling with the primary amino group to form the amide bond which links the acyl side chain to the appropriate nucleus. Suitable activated derivatives, their methods of preparation, and their methods of use as acylating agents for a primary amine will be recognized by those skilled in the art. Preferred activated derivatives are: (a) an acid halide (e.g. an acid chloride), (b) an acid anhydride (e.g. an alkoxyformic acid anhydride or aryloxyformic acid anhydride) or (c) an activated ester (e.g. a 2,4,5-trichlorophenyl ester). Other methods for activating the carboxyl function include reaction of the carboxylic acid with a carbonyldiimide (e.g. N,N'-dicyclohexylcarbodiimide or N,N'-diisopropylcarbodiimide) to give a reactive intermediate which, because of instability, is not isolated, the reaction with the primary amine being carried out *in situ.*

If a particular amino acid contains an acylable functional group other than the amino group, it will be understood by those skilled in the art that such a group must be protected prior to reaction of the amino acid with the reagent used to attach the N-alkanoyl or N-alkenoyl gorup. Suitable protecting groups can be any group known in the art to be useful for the protection of a side chain functional group in peptide synthesis. Such groups are well known, and the selection of a particular protecting group and its method of use will be readily known to one skilled in the art [see, for example, "Protective Groups In Organic Chemistry", M. McOmie, Editor, Plenum Press, N.Y., 1973].

The compounds of formula III inhibit the growth of pathogenic fungi and are useful, therefore, for controlling the growth of fungi on environmental surfaces (as an antiseptic) or in treating infections caused by fungi. In particular, the compounds are active against *Candida albicans* and are, thus, especially useful for treating candidosis. The activity of the compounds can be assessed in standard microbiological test procedures, such as *in vitro* in agar-plate disc-diffusion tests or in agar dilution tests, or *in vivo* in tests in mice infected with *C. albicans*. The compounds are also active against *Trichophyton mentagrophytes* (a dermatophytic organism), *Saccharomyces pastorianus*, and *Neurospora crassa*.

Certain compounds (as shown in Reference Example 19, Table VIII) give significant blood levels upon oral administration in mice.

When given to a dog by intravenous administration at a dosage level of 100 mg/kg per day for five days, the compound of formula III wherein $R^5$ is *p*-(*n*-octyloxy)benzoyl showed no outward signs of toxicity, although temporarily increased serum glutamic pyruvic transaminase (SGPT) levels were observed.

When used systemically, the dosage of the compounds of formula III will vary according to the particular compound being used, the severity and nature of the infection, and the physical condition of the subject being treated. Therapy should be initiated at low dosages, and the dosage should be increased until the desired antifungal effect is obtained. The compounds can be administered intravenously or intramuscularly by injection in the form of a sterile aqueous solution or suspension to which may be added, if desired, various conventional pharmaceutically acceptable preserving, buffering, solubilizing or suspending agents. Other additives, such as saline or glucose, may be added to make the solutions isotonic. The proportions and nature of such additives will be apparent to those skilled in the art.

Certain compounds of formula III give significant blood levels after oral administration (see Reference Example 19, Table VIII) and can be administered systematically by the oral route. For oral use, such compounds can be administered in combination with pharmaceutically acceptable carriers or excipients in the form of capsules, tablets or powders. The nature and proportion of such carriers or excipients will be recognized by those skilled in the art.

When used to treat vaginal *Candida* infections, the compounds of formula III can be administered in combination with pharmaceutically acceptable conventional excipients suitable for intravaginal use. Formulations adapted for intravaginal administration will be known to those skilled in the art.

In order to illustrate the operation of this invention more fully, the following examples are provided.

## Preparation 1

Fermentation of *Actinoplanes utahensis*

A stock culture of *Actinoplanes utahensis* NRRL 12052 is prepared and maintained on an agar slant. The medium used to prepare the slant is selected from one of the following:

### MEDIUM A

| Ingredient | Amount |
|---|---|
| Pre-cooked oatmeal | 60.0 g |
| Yeast | 2.5 g |
| $K_2HPO_4$ | 1.0 g |
| Czapek's mineral stock* | 5.0 ml |
| Agar | 25.0 g |
| Deionized water | q.s. to 1 liter |

pH before autoclaving is about 5.9; adjust to pH 7.2 by addition of NaOH; after autoclaving, pH is about 6.7.

*Czapek's mineral stock has the following composition:

| Ingredient | Amount |
|---|---|
| $FeSO_4 \cdot 7H_2O$ (dissolved in 2 ml conc HCl) | 2 g |
| KCl | 100 g |
| $MgSO_4 \cdot 7H_2O$ | 100 g |
| Deionized water | q.s. to 1 liter |

MEDIUM B

| Ingredient | Amount |
|---|---|
| Potato dextrin | 5.0 g |
| Yeast extract | 0.5 g |
| Enzymatic hydrolysate of casein* | 3.0 g |
| Beef extract | 0.5 g |
| Glucose | 12.5 g |
| Corn starch | 5.0 g |
| Meat peptone | 5.0 g |
| Blackstrap molasses | 2.5 g |
| $MgSO_4 \cdot 7H_2O$ | 0.25 g |
| $CaCO_3$ | 1.0 g |
| Czapek's mineral stock | 2.0 ml |
| Agar | 20.0 g |
| Deionized water | q.s. to 1 liter |

*N—Z—Amine A, Humko Sheffield Chemical, Lyndhurst, N.J.

The slant is inoculated with *Actinoplanes utahensis* NRRL 12052, and the inoculated slant is incubated at 30°C for about 8 to 10 days. About 1/2 of the slant growth is used to inoculate 50 ml of a vegetative medium having the following composition:

| Ingredient | Amount |
|---|---|
| Pre-cooked oatmeal | 20.0 g |
| Sucrose | 20.0 g |
| Yeast | 2.5 g |
| Distiller's Dried Grain* | 5.0 g |
| $K_2HPO_4$ | 1.0 g |
| Czapek's mineral stock | 5.0 ml |
| Deionized water | q.s. to 1 liter |

Adjust to pH 7.4 with NaOH; after autoclaving, pH is about 6.8.

*National Distillers Products Co., 99 Park Ave., New York, N.Y.

The inoculated vegetative medium is incubated in a 250-ml wide-mouth Erlenmeyer flask at 30°C for about 72 hours on a shaker rotating through an arc two inches in diameter at 250 RPM.

This incubated vegetative medium may be used directly to inoculate a second-stage vegetative medium. Alternatively and preferably, it can be stored for later use by maintaining the culture in the vapor phase of liquid nitrogen. The culture is prepared for such storage in multiple small vials as follows: In each vial is placed 2 ml of incubated vegatative medium and 2 ml of a glycerol-lactose solution [see W. A. Dailey and C. E. Higgens, "Preservation and Storage of Microorganisms in the Gas Phase of Liquid Nitrogen, *Cryobiol 10*, 364—367 (1973) for details]. The prepared suspensions are stored in the vapor phase of liquid nitrogen.

A stored suspension (1 ml) thus prepared is used to inoculate 50 ml of a first-stage vegetative medium (having the composition earlier described). The inoculated first-stage vegetative medium is incubated as above-described.

In order to provide a larger volume of inoculum, 10 ml of the incubated first-stage vegetative medium is used to inoculate 400 ml of a second-stage vegetative medium having the same composition as the first-stage vegetative medium. The second-stage medium is incubated in a two-liter wide-mouth Erlenmeyer flask at 30°C for about 48 hours on a shaker rotating through an arc two inches in diameter at 250 RPM.

Incubated second-stage vegetative medium (800 ml), prepared as above-described, is used to inoculate 100 liters of sterile production medium selected from one of the following:

MEDIUM I

| Ingredient | Amount (g/L) |
|---|---|
| Peanut meal | 10.0 |
| Soluble meat peptone | 5.0 |
| Sucrose | 20.0 |
| $KH_2PO_4$ | 0.5 |
| $K_2HPO_4$ | 1.2 |
| $MgSO_4 \cdot 7H_2O$ | 0.25 |
| Tap water | q.s. to 1 liter |

The pH of the medium is about 6.9 after sterilization by autoclaving at 121°C for 45 minutes at about 16—18 psi.

MEDIUM II

| Ingredient | Amount (g/L) |
|---|---|
| Sucrose | 30.0 |
| Peptone | 5.0 |
| $K_2HPO_4$ | 1.0 |
| KCl | 0.5 |
| $MgSO_4 \cdot 7H_2O$ | 0.5 |
| $FeSO_4 \cdot 7H_2O$ | 0.002 |
| Deionized water | q.s. to 1 liter |

Adjust to pH 7.0 with HCl; after autoclaving, pH is about 7.0.

MEDIUM III

| Ingredient | Amount (g/l) |
|---|---|
| Glucose | 20.0 |
| $NH_4Cl$ | 3.0 |
| $Na_2SO_4$ | 2.0 |
| $ZnCl_2$ | 0.019 |
| $MgCl_2 \cdot 6H_2O$ | 0.304 |
| $FeCl_3 \cdot 6H_2O$ | 0.062 |
| $MnCl_2 \cdot 4H_2O$ | 0.035 |
| $CuCl_2 \cdot 2H_2O$ | 0.005 |
| $CaCO_3$ | 6.0 |
| $KH_2PO_4$* | 0.67 |
| Tap water | q.s. to 1 liter |

*Sterilized separately and added aseptically
Final pH about 6.6.

The inoculated production medium is allowed to ferment in a 165-liter fermentation tank at a temperature of about 30°C for about 42 hours. The fermentation medium is stirred with conventional agitators at about 200 RPM and aerated with sterile air to maintain the dissolved oxygen level above 30% of air saturation at atmospheric pressure.

Preparation 2
Preparation of the A—42355 Antibiotic Complex

A. Shake-Flask Fermentation

A culture of *Aspergillus nidulans* var. *roseus* NRRL 11440 is prepared and maintained on an agar slant prepared with medium having the following composition:

| Ingredient | Amount |
|---|---|
| Glucose | 5 g |
| Yeast extract | 2 g |
| $CaCO_3$ | 3 g |
| Vegetable juice* | 200 ml |
| Agar** | 20 g |
| Deionized water | q.s. to 1 liter |

(initial pH 6.1)
*V—8 Juice, Campbell Soup Co., Camden, N.J.
**Meer Corp.

The slant is inoculated with *Aspergillus nidulans* var. *roseus* NRRL 11440, and the inoculated slant is incubated at 25°C. for about seven days. The mature slant culture is covered with water and scraped with a sterile loop to loosen the spores. The resulting suspension is further suspended in 10 ml of sterile deionized water.

One ml of the suspended slant growth is used to inoculate 55 ml of vegetative medium in a 250-ml flask. The vegetative medium has the following composition:

| Ingredient | Amount |
|---|---|
| Sucrose | 25 g |
| Blackstrap molasses | 36 g |
| Corn-steep liquor | 6 g |
| Malt extract | 10 g |
| $K_2HPO_4$ | 2 g |
| Enzymatic hydrolysate of casein* | 10 g |
| Tap water | 1100 ml |

(initial pH 6.5—6.7

*N—Z—Case, Humko Sheffield Chemical, Lyndhurst, N.J.

The inoculated vegetative medium is incubated at 25°C. for 48 hours at 250 rpm on a rotary-type shaker. After 24 hours, the medium is homogenized for one minute at low speed in a blender (Waring type) and then returned to incubation for the remaining 24 hours. Alternatively, the inoculated vegetative medium can be incubated for 48 hours and then homogenized for 15 seconds at low speed.

This incubated vegetative medium may be used to inoculate shake-flask fermentation culture medium or to inoculate a second-stage vegetative medium. Alternatively, it can be stored for later use by maintaining the culture in the vapor phase of liquid nitrogen. The culture is prepared for such storage in multiple small vials as follows:

The vegetative cultures are mixed volume/volume with a suspending solution having the following composition:

| Ingredient | Amount |
|---|---|
| Glycerol | 20 ml |
| Lactose | 10 g |
| Deionized water | q.s. to 100 ml |

The prepared suspensions are distributed in small sterile screw-cap tubes (4 ml per tube). These tubes are stored in the vapor phase of liquid nitrogen.

A stored suspension thus prepared can be used to inoculate either agar slants or liquid seed media. Slants are incubated at 25°C. in the light for 7 days.

B. Tank Fermentation

In order to provide a larger volume of inoculum, 10 ml of incubated first-stage vegetative culture is used to inoculate 400 ml of a second-stage vegetative growth medium having the same composition as that of the vegetative medium. The second-stage medium is incubated in a two-liter wide-mouth Erlenmeyer flask at 25°C. for 24 hours on a shaker rotating through an arc two inches in diameter at 250 rpm.

Incubated second-stage medium (800 ml), prepared as above described, is used to inoculate 100 liters of sterile production medium selected from one of the following:

MEDIUM IV

| Ingredient | Amount |
|---|---|
| $ZnSO_4 \cdot 7H_2O$ | 0.00455 g/L |
| Soluble meat peptone* | 30.5 g/L |
| Soybean meal | 15.5 g/L |
| Tapioca dextrin** | 2.0 g/L |
| Blackstrap molasses | 10.5 g/L |
| Enzymatic hydrolysate of casein*** | 8.5 g/L |
| $Na_2HPO_4$ | 4.5 g/L |
| $MgSO_4 \cdot 7H_2O$ | 5.5 g/L |
| $FeSO_4 \cdot 7H_2O$ | 0.1 g/L |
| Cottonseed oil | 40.0 ml |
| (Antifoam)**** | 1.0 ml |
| Tap water | 1000.0 ml |

(initial pH 6.8—7.0)
*O.M. Peptone, Amber Laboratories, Juneau, Wisc.
**Stadex 11, A. E. Staley Co., Decatur, Ill.
***N—Z—Amine A, Humko Sheffield Chemical, Lyndhurst, N.J.
****P2000, Dow Corning, Midland, Michigan

MEDIUM V

| Ingredient | Amount |
|---|---|
| Glucose | 2.5% |
| Starch | 1.0% |
| Soluble meat peptone* | 1.0% |
| Blackstrap molasses | 1.0% |
| $CaCO_3$ | 0.2% |
| $MgSO_4 \cdot 7H_2O$ | 0.05% |
| Enzymatic hydrolysate of casein** | 0.4% |
| (Antifoam)*** | 0.02% |
| Tap water | q.s. to volume |

*O.M. Peptone
**N—Z—Amine A
***Antifoam "A", Dow Corning

The inoculated production medium is allowed to ferment in a 165-liter fermentation tank at a temperature of 25°C. for about 7 days. The fermentation medium is aerated with sterile air, maintaining the dissolved oxygen level above approximately 50 percent of air saturation.

C. Third-Stage Vegetative Medium

Whenever the fermentation is carried out in tanks larger than those used for 100-liter fermentation, it is recommended that a third-stage vegetative culture be used to seed the larger tank. A

preferred third-stage vegetative medium has the following composition:

| Ingredient | Amount |
| --- | --- |
| Sucrose | 25 g |
| Blackstrap molasses | 25 g |
| Corn-steep liquor | 6 g |
| Enzymatic hydrolysate of casein* | 10 g |
| Malt extract | 10 g |
| $K_2HPO_4$ | 2 g |
| Tap water | 1000 ml |

(initial pH 6.1)
*N—Z—Case

Preparation 3

Separation of the A—42355 Antibiotic Complex

Whole fermentation broth (4127 liters), obtained by the method described in Preparation 2 using production medium V, is stirred thoroughly with methanol (4280 liters) for one hour and then is filtered, using a filter aid (Hyflo Super-cel, a diatomaceous earth, Johns-Manville Products Corp.). The pH of the filtrate is adjusted to pH 4.0 by the addition of 5 N HCl. The acidified filtrate is extracted twice with equal volumes of chloroform. The chloroform extracts are combined and concentrated under vacuum to a volume of about 20 liters. This concentrate is added to about 200 liters of diethyl ether to precipitate the A—42355 complex. The precipitate is separated by filtration to give 2775 g of the A—42355 complex as a gray-white powder.

Preparation 4

Isolation of A—30912 Factor A

A—42355 antibiotic complex (1 g), prepared as described in Preparation 3, is dissolved in 7 ml of methanol:water:acetonitrile (7:2:1). This solution is filtered and introduced onto a 3.7-cm I.D. x 35-cm glass column [Michel-Miller High Performance Low Pressure (HPLPLC) Chromatography Column, Ace Glass Incorporated, Vineland, NJ 08360] packed with LP—1/$C_{18}$ silica gel reversed-phase resin (10—20 $\mu$m), prepared as described in Preparation 10, through a loop with the aid of a valve system. The column is packed in methanol:water:acetonitrile (7:2:1) by the slurry-packing procedure described in Preparation 11. An F.M.I. pump with valveless piston design (maximum flow 19.5 ml/minute) is used to move the solvent through the column at a flow rate of 9 ml/minute at *ca.* 100 psi (6.9 bar), collecting fractions every minute. Elution of the antibiotic is monitored at 280 nm by using a UV monitor (ISCO Model UA—5, Instrument Specialist Co., 4700 Superior Ave., Lincoln, Nebraska 68504) with an optical unit (ISCO type 6).

Fractions (about 112—140) are combined and added to 20 ml of water. The pH of this solution is adjusted to pH 4.0 with *N* HCl. The resulting solution is extracted twice with equal volumes of chloroform. The two chloroform extracts are combined and concentrated under vacuum to give an oil. The oil is dissolved in tertiary butanol, and this solution is lyophilized to give 524 mg of A—42355 factor A (A—30912 factor A; A—22082).

Preparation 5

Isolation of A—30912 Factor B

A—42355 complex is separated as described in Preparation 3 except that the concentrated chloroform extracts (285 L) are chromatographed over a silica-gel column (150 L of Grace silica-gel, grade 62) at a flow rate of 2 L/min. The column is washed with chloroform (200 L), eluted with acetonitrile (500 L), and then continuously eluted with acetonitrile:water (98:2) at a flow rate of 1 L/min. Fractions having a volume of approximately 200 L are collected and analyzed individually for biological activity. The bioassay is performed by a paper-disc assay on agar plates seeded with *Candida albicans*. Fractions 77 through 103 (1365 L) are combined and concentrated under vacuum. The concentrated solution (4.5 L) contains a precipitate which is removed by filtration to give 119 g of factor B-enriched A—42355 complex. The filtrate is concentrated to dryness; the residue obtained is redissolved in an appropriate volume of methanol. The methanol solution is added to diethyl ether (10 volumes) to precipitate the factor-B-containing antibiotic complex. This precipitate is also separated by

filtration and dried to give an additional 24 g of factor-B-enriched A—42355 complex as a gray powder.

Factor-B-enriched A—42355 complex thus obtained (1.0 g) is dissolved in 8 ml of methanol:water:acetonitrile (7:2:1). This solution is filtered and introduced onto a silica-gel column (3.7-cm I.D. x 33-cm Michel-Miller Column) through a loop with the aid of a valve system. The column is packed with LP—1/$C_{18}$ silica-gel reversed-phase resin (10—20 μm), prepared as described in Preparation 10, in methanol:water:acetonitrile (7:2:1) through a loop with the aid of a valve system. The slurry packing procedure described in Preparation 11 is used. The solvent is moved through the column at a flow rate of 10 ml/min at *ca.* 10 psi (6.9 bar), using an F.M.I. pump with valveless piston design. One fraction is collected every minute. Elution of the antibiotic is monitored using a UV monitor at 280 nm as in Example 1. Fractions 102—110 are combined and concentrated under vacuum to give an oil. The oil is dissolved in a small volume of *tert*-butanol and lyophilized to give 22 mg of A—30912 factor B.

Preparation 6

Isolation of A—30912 Factor D

Concentrated chloroform extracts from two fermentation runs (3800 L and 4007 L) obtained by the method described in Preparation 3 were combined and chromatographed on a silica-gel column (Grace, grade 62). The column was washed with chloroform and then was eluted with acetonitrile and acetonitrile:water (98:2). Fractions having a volume of approximately 200 L were collected and analyzed for biological activity by paper-disc assay on agar seeded with *Candida albicans.* Fractions having activity (850 L) were combined and concentrated under vacuum. The concentrated solution (0.7 L) was added to diethyl ether (10 volumes) to precipitate the factor D-enriched A—42355 complex. This precipitate was removed by filtration and dried to give 32 g, of factor D-enriched A—42355 complex as a gray powder.

Factor D-enriched A—42355 complex thus obtained (1.0 g,) was dissolved in 5 ml. of methanol:water:acetonitrile (7:2:1). This solution was filtered and introduced onto a silica-gel column (3.7-cm I.D. x 30-cm Michel-Miller Column) through a loop with the aid of a valve system. The column was packed with LP—1/$C_{18}$ silica-gel reversed-phase resin (10—20 μm; prepared as described in Preparation 10). Packing was accomplished in methanol:water:acetonitrile (7:2:1) by the slurry-packing procedure described in Preparation 11. The solvent was moved through the column at a flow rate of 8 ml/min at ca. 45 psi (3.1 bar) using an F.M.I. pump with valveless piston design. One fraction was collected every 2 minutes. Elution of the antibiotic was monitored at 280 nm by using a UV monitor (ISCO Model UA—5) with an optical unit (ISCO Type 6). Fractions 96—108 were combined and concentrated under vacuum to give an oil. This oil was dissolved in a small volume of *tert*-butanol and lyophilized to give 89 mg of A—30912 factor D.

Preparation 7

Isolation of A—30912 Factor H

A—42355 antibiotic complex (5.0 g), prepared as described in Preparation 3, was dissolved in 35 ml of methanol:water:acetonitrile (7:2:1); the resulting solution was filtered and introduced onto a 3.7-cm I.D x 42-cm glass column (Michel-Miller Column) through a loop with the aid of a valve system. The column was packed with LP—1/$C_{18}$ silica gel reversed phase resin (10—20 μm) in methanol:water:acetonitrile (7:2:1) as described in Preparation 11. The solvent was moved through the column at a flow rate of 13 ml/min at *ca.* 120 psi (8.3 bar), using an F.M.I. pump with valveless piston design and collecting one fraction every two minutes. Elution of the antibiotic was monitored by UV at 280 nm. Fractions 112—132 were combined with fractions 106—117 from a second similar purification. The combined fractions were concentrated under vacuum to an oil. The oil was dissolved in a small amount of *tert*-butanol and lyophilized to give 173 mg of crude A—30912 factor H.

The crude A—30912 factor H (150 mg) was dissolved in 8 ml of methanol:water:acetonitrile (7:2:1); the resulting solution was filtered and introduced onto a 2.0-cm I.D. x 32-cm glass column, as described above. The solvent was moved through the column at a flow rate of 8 ml/min at *ca.* 80 psi (5.5 bar) collecting one fraction every three minutes. Elution of the antibiotic was monitored at 280 nm. Fractions 17 and 18 were combined and concentrated under vacuum to give an oil. The oil was dissolved in a small volume of *tert*-butanol and lyophilized to give 29 mg of A—30912 factor H.

Preparation 8

Preparation of Antibiotic S 31794/F—1

Antibiotic S 31794/F—1 is produced by submerged culture of *Acrophialophora limonispora* NRRL 8095 with stirring, shaking, and/or aeration at pH 3—8, preferably pH 5—7, and at 15—30°C., preferably at 18—27°C., for from 48 to 360 hours, preferably from 120 to 288 hours.

Antibiotic S 31794/F—1 is isolated by treating the culture broth (90 L) with ethyl acetate:isopropanol (4:1, 90 L) and homogenizing for 30 minutes at room temperature. The organic phase is separated and evaporated under vacuum at about 40°C. The residue thus obtained is chromatographed on a 10-fold amount of silica gel, using $CHCl_3$:$CH_3OH$ (95:5 to 60:40). Fractions

which have antifungal activity are combined and chromatographed on a 100-fold amount of "Sephadex LH—20" (Sephadex is a registered trademark) with methanol. Fractions from the Sephadex column which have antifungal activity are combined and rechromatographed on a 100-fold amount of silica gel (0.05—0.2 mm) with a $CHCl_3$:$CH_3OH$:$H_2O$ (71:25:4) solvent system. The fractions eluted which have antifungal activity are combined and evaporated under vacuum to give crude antibiotic S 31794/F—1. This product is dissolved in small amounts of methanol and precipitated with diethyl ether to give S 31794/F—1 as a white amorphous powder, mp 178—180°C. (dec.) after drying in high vacuum at 25—30°C. Crystallization from a 10-fold amount of ethyl acetate:methanol:water (80:12:8) gives crystalline S 31794/F—1, mp 181—183°C. (dec) after drying in high vacuum at 20°C.

Preparation 9

Isolation of Antibiotic S 31794/F—1

Crude antibiotic S 31794/F—1, obtained as described in Preparation 8 after chromatography over Sephadex, is introduced onto a silica-gel column (Michel-Miller Column) through a loop with the aid of a valve system. The column is packed with LP—1/$C_{18}$ silica-gel reversed-phase resin (10—20 $\mu$m), prepared as described in Preparation 10, in chloroform:methanol:water (71:25:4) through a loop with the aid of a valve system. The slurry packing procedure described in Preparation 11 is used. The solvent is moved through the column using an F.M.I. pump with valveless piston design. Elution of the antibiotic is monitored using a UV monitor at 280 nm. Fractions having antifungal activity are combined and concentrated under vacuum to give antibiotic S 31794/F—1.

Preparation 10

Preparation of Silica Gel/$C_{18}$ Reversed Phase Resin

*Step 1: Hydrolysis*

LP—1 silica gel (1000 g from Quantum Corp., now Whatman) is added to a mixture of concentrated sulfuric acid (1650 ml) and concentrated nitric acid (1650 ml) in a 5—L round-bottom flask and shaken for proper suspension. The mixture is heated on a steam bath overnight (16 hours) with a water-jacketed condenser attached to the flask.

The mixture is cooled in an ice bath and carefully filtered using a sintered-glass funnel. The silica gel is washed with deionized water until the pH is neutral. The silica gel is then washed with acetone (4 L) and dried under vacuum at 100°C. for 2 days.

*Step 2: First Silylation*

The dry silica gel from Step 1 is transferred to a round-bottom flask and suspended in toluene (3.5 L). The flask is heated on a steam bath for 2 hours to azeotrope off some residual water. Octadecyltrichlorosilane (321 ml, Aldrich Chemical Company) is added, and the reaction mixture is refluxed overnight (16 hours) with slow mechanical stirring at about 60°C. Care is taken so that the stirrer does not reach near the bottom of the flask. This is to prevent grinding the silica gel particles.

The mixture is allowed to cool. The silanized silica gel is collected, washed with toluene (3 L) and acetone (3 L), and then air-dried overnight (16—20 hours). The dried silica gel is suspended in 3.5 L of acetonitrile:water (1:1) in a 5—L flask, stirred carefully at room temperature for 2 hours, filtered, washed with acetone (3 L) and air-dried overnight.

*Step 3: Second Silylation*

The procedure from the first silylation is repeated using 200 ml of octadecyltrichlorosilane. The suspension is refluxed at 60°C. for 2 hours while stirring carefully. The final product is recovered by filtration, washed with toluene (3 L) and methanol (6 L), and then dried under vacuum at 50°C. overnight (16—20 hours).

Preparation 11

Slurry Packing Procedure for Michel-Miller Columns

*General Information*

A. Analytical or preparative columns can be packed by this procedure.

B. Silica gels and silica gel reversed phase packings (e.g., Quantum LP—1, particle size 10—20 $\mu$m; LiChroprep RP—8 and RP—18, particle size 25—40 microns) are recommended. However, other silica gels (e.g., Shandons ODS Hypersil, particle size 5 mm) as well as other types of resins have been packed successfully by this procedure.

C. Generally a pressure of less than 200 psi (14 bar) and flow rates between 5—40 ml/minute are required for this slurry packing technique; this is dependent on column volume and size. Packing pressure should exceed pressure used during actual separation by 30—50 psi (2.1—3.5 bar); this will assure no further compression of the adsorbent during separation runs. Columns packed by this procedure with reversed-phase silica gel can be operated for several years without loss of efficiency.

D. Sudden decrease in pressure may cause cracks or channels to form in the packing material, which would greatly reduce column efficiency. Therefore, it is important to let the pressure drop slowly to zero whenever the pump has been turned off.

E. Approximate volume of columns (Ace Glass Cat. No., unpacked): 5795—04, 12 ml; 5795—10, 110 ml; 5795—16, 300 ml; 5795—24, 635 ml; and 5796—34, 34 ml.

F. The time required to pack a glass column will vary from minutes to several hours depending on column size and experience of the scientist.

*Specific information*

1. Connect glass column to a reservoir column via coupling (volume of reservoir column should be twice that of the column). Place both columns in vertical positions (reservoir column above).
2. Weigh out packing material (*ca.* 100 g for 200 ml column).
3. Add *ca.* five volumes of solvent to packing material; use a mixture of 70—80% methanol and 20—30% water.
4. Shake well until all particles are wetted, let stand overnight or longer to assure complete soaking of particles by solvent. Decant supernatant liquid.
5. Slurry the resin with sufficient solvent to fill reservoir column. Pour swiftly into reservoir. NOTE: The column must be pre-filled with the same solvent and the reservoir column should be partly filled with solvent before slurry is poured. The use of larger slurry volumes may also provide good results; however, this will require (a) larger reservoir or (b) multiple reservoir fillings during the packing procedure.
6. Close reservoir with the Teflon (registered trademark) plug beneath the column (see Figure 1 of U.S. Patent 4,131,547, plug No. 3); connect to pump; and immediately start pumping solvent through system at maximum flow rate if Ace Cat. No. 13265—25 Pump or similar solvent-delivery system is used (ca. 20 ml/minute).
7. Continue until column is completely filled with adsorbent. Pressure should not exceed maximum tolerance of column during this operation (*ca.* 200 psi (14 bar) for large columns and 300 psi (21 bar) for analytical columns). In most cases, pressure less than 200 psi (14 bar) will be sufficient.
8. Should pressure exceed maximum values, reduce flow-rate; pressure will drop.
9. After column has been filled with adsorbent, turn off pump; let pressure drop to zero; disconnect reservoir; replace reservoir with a pre-column; fill pre-column with solvent and small amount of adsorbent; and pump at maximum pressure until column is completely packed. For additional information, see general procedure.

Always allow pressure to decrease slowly after turning off pump — this will prevent formation of any cracks or channels in the packing material.

10. Relieve pressure and disconnect pre-column carefully. With small spatula remove a few mm (2—4) of packing from top of column; place 1 or 2 filter(s) in top of column; gently depress to top of packing material; and place Teflon plug on top of column until seal is confirmed. Connect column to pump, put pressure on (usually less than 200 psi (14 bar)) and observe through glass wall on top of column if resin is packing any further. If packing material should continue to settle (this may be the case with larger columns), some dead space or channelling will appear and step 9 should be repeated.

## Preparation 12

Preparation of Tetrahydro-A—30912A

A—30912 factor A is dissolved in ethanol. $PtO_2$ in absolute ethanol is reduced to form Pt, which in turn is used to reduce the A—30912 factor A catalytically, using hydrogenation under positive pressure until the reaction is complete (about 2—3 hours). The reaction mixture is filtered and concentrated under vacuum. The residue is dissolved in a small amount of *tert*-butanol and lyophilized to give tetrahydro-A—30912A.

## Preparation 13

Preparation of Tetrahydro-A—30912B

A—30912 factor B is dissolved in ethanol. $PtO_2$ in absolute ethanol is reduced to form Pt, which in turn is used to reduce the A—30912 factor B catalytically, using hydrogenation under positive pressure until the reaction is complete (about 2—3 hours). The reaction mixture is filtered and concentrated under vacuum. The residue is dissolved in a small amount of *tert*-butanol and lyophilized to give tetrahydro-A—30912B.

## Preparation 14

Preparation of Tetrahydro-A—30912D

A—30912 factor D is dissolved in ethanol. $PtO_2$ in absolute ethanol is reduced to form Pt, which in turn is used to reduce the A—30912 factor D catalytically, using hydrogenation under positive pressure until the reaction is complete (about 2—3 hours). The reaction mixture is filtered and concentrated under vacuum. The residue is dissolved in a small amount of *tert*-butanol and lyophilized to give tetrahydro-A—30912D.

Preparation 15

Preparation of Tetrahydro-A—30912H

A—30912 factor H is dissolved in ethanol. $PtO_2$ in absolute ethanol is reduced to form Pt, which in turn is used to reduce the A—30912 factor H catalytically, using hydrogenation under positive pressure until the reaction is complete (about 2—3 hours). The reaction mixture is filtered and concentrated under vacuum. The residue is dissolved in a small amount of *tert*-butanol and lyophilized to give tetrahydro-A—30912H.

Example 1

A. Deacylation of A—30912 Factor A

A fermentation of *A. utahensis* is carried out as described in Preparation 1, using slant medium A and production medium I and incubating the production medium for about 42 hours. A—30912 factor A (340 g of crude substrate which contained about 19.7 g. of A—30912 factor A, dissolved in 1.5 L ethanol) is added to the fermentation medium.

Deacylation of A—30912 factor A is monitored by assay against *Candida albicans.* The fermentation is allowed to continue until deacylation is complete as indicated by disappearance of activity vs. *C. albicans.*

B. Isolation of A—30912A Nucleus

Whole fermentation broth (100 liters), obtained as described in Sect. B and containing nucleus from about 20 g of A—30912 factor A, is filtered. The mycelial cake is discarded. The clear filtrate thus obtained (about 93 liters) is passed through a column containing 4.5 liters of HP—20 resin (DIAION High Porous Polymer, HP—Series, Mitsubishi Chemical Industries Limited, Tokyo, Japan) at a rate of 200 ml/minute. The effluent thus obtained is discarded. The column is then washed with up to eight column volumes of deionized water at pH 6.5—7.5 to remove residual filtered broth. This wash water is discarded. The column is then eluted with a water:methanol (7:3) solution (85 liters) at a rate of 200—300 ml/minute.

Elution is monitored using the following procedure: Two aliquots are taken from each eluted fraction. One of the aliquots is concentrated to a small volume and is treated with an acid chloride such as myristoyl chloride, using a procedure such as that described in Reference Example 1. This product and the other (untreated) aliquot are assayed for activity against *Candida albicans.* If the untreated aliquot does not have activity and the acylated aliquot does have activity, the fraction contains A—30912A nucleus. The eluate containing the A—30912A nucleus is concentrated under vacuum to a small volume and lyophilized to give approximately 97 grams of crude nucleus.

C. Purification of A—30912A Nucleus by Reversed-Phase Liquid Chromatography

Crude A—30912A nucleus (25 grams), obtained as described in Section B, is dissolved in 300 ml of water:acetonitrile:acetic acid:pyridine (96:2:1:1). This solution is chromatographed on a 4-liter stainless-steel column (8 cm x 80 cm) filled with Lichroprep RP—18, particle size 25—40 $\mu$m (MC/B Manufacturing Chemists, Inc. E/M. Cincinnati, OH). The column is part of a Chromatospac Prep 100 unit (Jobin Yvon, 16—18 Rue du Canal 91160 Longjumeau, France). The column is operated at a pressure of 90—100 psi (6.2—6.9 bar), giving a flow rate of about 60 ml/minute, using the same solvent. Separation is monitored at 280 nm using a UV monitor (ISCO Absorption Monitor UA—5, Instrumentation Specialties Co., 4700 Superior Ave., Lincoln, Nebraska 68504) with an optical unit (ISCO Type 6). Fractions having a volume of about 500 ml are collected each minute.

On the basis of absorption at 280 nm, fractions containing A—30912A nucleus are combined, evaporated under vacuum and lyophilized to give 2.6 grams of nucleus. The amount of solvent required to complete this chromatographic separation process varies from 7—8 liters.

D. Characteristics of A30912A nucleus

(a) Empirical formula: $C_{34}H_{51}N_7O_{15}$.

(b) Molecular weight: 797.83.

(c) White amorphous solid, soluble in water, dimethylformamide, dimethylsulfoxide, and methanol; insoluble in chloroform, toluene, and diethylether.

(d) Infrared absorption spectrum (KBr disc.)

Shows absorption maxima at:

3340 broad (OH, H-bonded); 2970, 2930, and 2890 (CH stretch, aliphatic $CH_3$, $CH_2$, CH groups) 1660 and 1625 (several carbonyls C=O); 1510—1550; 1430—1450 (CH wag); 1310—1340; 1230—1260; 1080; 835, 650 broad, and 550 broad $cm^{-1}$.

(e) Electrometric titration in 66% aqueous dimethylformamide indicates the presence of a titratable group with a $pK_a$ value of about 7.35 (initial pH 7.32).

(f) HPLC retention time (K'):11.52 min. under following conditions.

Column: 4 x 300 mm

Packing: silica gel/$C_{18}$

Solvent: ammonium acetate:acetonitrile:water (1:2:97)

Flow Rate: 3 ml/min
Pressure: 2500 psi (172 bar)
Detector: variable wavelength UV at 230 nm
Sensitivity: 0—0.4 A.U.F.S.

Example 2

A—30912A nucleus is prepared and purified by the process of Example 1 except that tetrahydro-A—30912A is used as the substrate.

Example 3

A—30912A nucleus is prepared and purified by the process of Example 1 except that aculeacin A is used as the substrate.

Example 4

A. Deacylation of A—30912 Factor B

A fermentation of *A. utahensis* is carried out as described in Preparation 1, using production medium I. After the culture is incubated for about 48 hours, A—30912 factor B, dissolved in a small amount of methanol, is added to the fermentation medium.

Deacylation of A—30912 factor B is monitored by a paper-disc assay against *Candida albicans* or *Neurospora crassa.* The fermentation is allowed to continue until deacylation is complete as indicated by disappearance of activity.

B. Isolation of A—30912B Nucleus

Whole fermentation broth, obtained as described in Sect. B is filtered. The mycelial cake is discarded. The clear filtrate thus obtained is passed through a column containing HP—20 resin (DIAION High Porous Polymer, HP—Series, Mitsubishi Chemical Industries Limited, Tokyo, Japan). The effluent thus obtained is discarded. The column is then washed with up to eight column volumes of deionized water at pH 6.5—7.5 to remove residual filtered broth. This wash water is discarded. The column is then eluted with a water:methanol (7:3) solution. Elution is monitored using the following procedure: Two aliquots are taken from each eluted fraction. One of the aliquots is concentrated to a small volume and is treated with an acid chloride such as myristoyl chloride, using the procedure described in Preparation 11. This product and the other (untreated) aliquot are assayed for activity against *Candida albicans*. If the untreated aliquot does not have activity and the acylated aliquot does have activity, the fraction contains A—30912B nucleus. The eluate containing A—30912B nucleus is concentrated under vacuum to a small volume and lyophilized to give crude nucleus.

C. Purification of A—30912B Nucleus by Reversed-Phase Liquid Chromatography

Crude A—30912B nucleus, obtained as described in Section B, is dissolved in water:acetonitrile:acetic acid:pyridine (96:2:1:1). This solution is chromatographed on a column filled with Lichroprep RP—18, particle size 25—40 $\mu$m (MC/B Manufacturing Chemists, Inc. E/M, Cincinnati, OH). The column is part of a Chromatospac Prep 100 unit (Jobin Yvon, 16—18 Rue du Canal 91160 Longjumeau, France). The column is operated at a pressure of 90—100 psi (6.2—6.9 bar), giving a flow rate of about 60 ml/minute, using the same solvent. Separation is monitored at 280 nm using a UV monitor (ISCO Absorption Monitor Model UA—5, Instrumentation Specialties Co., 4700 Superior Ave., Lincoln, Nebraska 68504) with an optical unit (ISCO Type 6).

On the basis of absorption at 280 nm, fractions containing A—30912B nucleus are combined, evaporated under vacuum and lyophilized to give purified A—30912B nucleus.

Example 5

A—30912B nucelus is prepared and purified by the method of Example 4 except that tetrahydro-A—30912B is used as the substrate.

Example 6

A. Deacylation of A—30912 Factor D

A fermentation of *A. utahensis* is carried out as described in Preparation 1, using production medium I. After the culture is incubated for about 48 hours, A—30912 factor D, dissolved in a small amount of methanol, is added to the fermentation medium.

Deacylation of A—30912 factor D is monitored by paper-disc assay against *Candida albicans* or *Neurospora crassa.* The fermentation is allowed to continue until deacylation is complete as indicated by disappearance of activity.

B. Isolation of A—30912D Nucleus

Whole fermentation broth, obtained as described in Sect. A is filtered. The mycelial cake is discarded. The clear filtrate thus obtained is passed through a column containing HP—20 resin (DIAION High Porous Polymer, HP—Series, Mitsubishi Chemical Industries Limited, Tokyo, Japan). The

effluent thus obtained is discarded. The column is then washed with up to eight column volumes of deionized water at pH 6.5—7.5 to remove residual filtered broth. This wash water is discarded. The column is then eluted with a water:methanol (7:3) solution. Elution is monitored using the following procedure: Two aliquots are taken from each eluted fraction. One of the aliquots is concentrated to a small volume and is treated with an acid chloride such as myristoyl chloride, using the procedure described in Reference Example 1. This product and the other (untreated) aliquot are assayed for activity against *Candida albicans*. If the untreated aliquot does not have activity and the acylated aliquot does have activity, the fraction contains A—30912D nucleus. The eluate containing A—30912D nucelus is concentrated under vacuum to a small volume and lyophilized to give crude nucleus.

C. Purification of A—30912D Nucleus by Reversed-Phase Liquid Chromatography

Crude A—30912D nucleus, obtained as described in Section B, is purified according to the procedure of Section C of Example 4.

On the basis of absorption at 280 nm, fractions containing A—30912D nucleus are combined, evaporated under vacuum and lyophilized to give purified A—30912D nucleus.

Example 7

A—30912D nucleus is prepared and purified by the method of Example 6 except that tetrahydro-A—30912D is used as the substrate.

Example 8

A. Deacylation of A—30912 Factor H

A fermentation of *A. utahensis* is carried out as described in Preparation 1, using production medium I. After the culture is incubated for about 48 hours, A—30912 factor H, dissolved in a small amount of methanol, is added to the fermentation medium.

Deacylation of A—30912 factor H is monitored by paper-disc assay against *Candida albicans* or *Neurospora crassa*. The fermentation is allowed to continue until deacylation is complete as indicated by disappearance of activity.

B. Isolation of A—30912H Nucleus

Whole fermentation broth, obtained as described in Sect. A, is filtered. The mycelial cake is discarded. The clear filtrate thus obtained is passed through a column containing HP—20 resin (DIAION High Porous Polymer, HP—Series, Mitsubishi Chemical Industries Limited, Tokyo, Japan). The effluent thus obtained is discarded. The column is then washed with up to eight column volumes of deionized water at pH 6.5—7.5 to remove residual filtered broth. This wash water is discarded. The column is then eluted with a water:methanol (7:3) solution. Elution is monitored using the following procedure: Two aliquots are taken from each eluted fraction. One of the aliquots is concentrated to a small volume and is treated with an acid chloride such as myristoyl chloride, using a procedure such as the one described in Reference Example 1. This product and the other (untreated) aliquot are assayed for activity against *Candida albicans.* If the untreated aliquot does not have activity and the acylated aliquot does have activity, the fraction contains A—30912H nucleus. The eluate containing A—30912H nucleus is concentrated under vacuum to a small volume and lyophilized to give crude nucleus.

C. Purification of A—30912H Nucleus by Reversed-Phase Liquid Chromatography

Crude A—30912H nucleus, obtained as described in Section B, is purified according to the process of Section C of Example 4.

On the basis of absorption at 280 nm, fractions containing A—30912H nucleus are combined, evaporated under vacuum and lyophilized to give purified A—30912H nucleus.

Example 9

A—30912H nucleus is prepared and purified by the method of Example 8 except that tetrahydro-A—30912H is used as the substrate.

Example 10

A—30912H nucleus is prepared and purified by the method of Example 8 except that A—30912 factor H is prepared from A—30912 factor A using the following procedure:

Antibiotic A—30912 factor A (19.6 mg) is dissolved in dimethylformamide (1 ml). Acidic methanol (3% HCl, 0.06 ml) is added to this solution. The resulting solution is stirred at room temperature overnight and then is evaporated to dryness under vacuum. The residue obtained is chromatographed by HPLPLC as described in Preparation 7, using reversed-phase silica gel (LP—1/$C_{18}$, prepared as described in Preparation 10) and $CH_3OH$:$H_2O$:$CH_3CN$ (7:2:1) as the eluting solvent to give 1.4 mg of A—30912 factor H (the compound of formula II wherein $R^1$ and $R^4$ are both hydroxy, $R^2$ is hydrogen, $R^3$ is methoxy and R is linoleoyl).

Example 11

The A—30912H-type nucleus of structure I wherein $R^3$ is ethoxy is prepared and purified by the method of Example 8, except that the compound of formula II wherein $R^3$ is ethoxy and R is linoleoyl is used as the substrate. The substrate is prepared by the procedure used in Example 10.

Example 12

The A—30912H-type nucleus having structure I wherein $R^3$ is *n*-propoxy is prepared and purified by the method of Example 8, except that the compound of formula II wherein $R^3$ is *n*-propoxy and R is linoleoyl is used as the substrate. The substrate is prepared as described in Example 10.

Example 13

The A—30912H nucleus having structure I wherein $R^3$ is isobutoxy is prepared and purified by the method of Example 8, except that the compound of formula II wherein $R^3$ is isobutoxy and R is linoleoyl is used as the substrate.

Example 14

The A—30912H nucleus of structure I wherein $R^3$ is *n*-pentyloxy is prepared and purified by the method of Example 8, except that the compound of formula II wherein $R^3$ is *n*-pentyloxy and R is linoleoyl is used as the substrate.

Example 15

The A—30912H-type nucleus of structure I wherein $R^3$ is *n*-hexyloxy is prepared and purified by the method of Example 8, except that the compound of formula II wherein $R^3$ is *n*-hexyloxy and R is linoleoyl is used as the substrate.

Example 16

The A—30912H-type nucleus having structure I wherein $R^3$ is ethoxy is prepared and purified by the method of Example 8, except that the compound having formula II wherein $R^3$ is ethoxy and R is stearoyl is used as the substrate.

Example 17

The A—30912H-type nucleus having structure I wherein $R^3$ is 2-ethyl-1-butoxy is prepared and purified by the method of Example 8, except that the compound of formula II wherein $R^3$ is 2-ethyl-1-butoxy and R is linoleoyl is used as the substrate.

Example 18

The A—30912H-type nucleus having structure I wherein $R^3$ is 3-methyl-1-butoxy is prepared and purified by the method of Example 8, except that the compound of formula II wherein $R^3$ is 3-methyl-1-butoxy and R is linoleoyl is used as the substrate.

Example 19

A. Deacylation of Antibiotic S 31794/F—1

A fermentation of *A. utahensis* is carried out as described in Preparation 1, using production medium I. After the culture is incubated for about 48 hours, antibiotic S 31794/F—1, dissolved in a small amount of methanol, is added to the fermentation medium.

Deacylation of S 31794/F—1 is monitored by paper-disc assay against *Candida albicans*. The fermentation is allowed to continue until deacylation is complete as indicated by disappearance of activity.

B. Isolation of S 31794/F—1 Nucleus

Whole fermentation broth, obtained as described in Sect. A is filtered. The mycelial cake is discarded. The clear filtrate thus obtained is passed through a column containing HP—20 resin (DIAION High Porous Polymer, HP-Series, Mitsubishi Chemical Industries Limited, Tokyo, Japan). The effluent thus obtained is discarded. The column is then washed with up to eight column volumes of deionized water at pH 6.5—7.5 to remove residual filtered broth. This wash water is discarded. The column is then eluted with a water: methanol (7:3) solution. Elution is monitored using the following procedure: Two aliquots are taken from each eluted fraction. One of the aliquots is concentrated to a small volume and is treated with an acid chloride such as myristoyl chloride, using the procedure described in Reference Example 1. This product and the other (untreated) aliquot are assayed for activity against *Candida albicans*. If the untreated aliquot does not have activity and the acylated aliquot does have activity, the fraction contains S 31794/F—1 nucleus. The eluate containing S 31794/F—1 nucleus is concentrated under vacuum to a small volume and lyophilized to give crude nucleus.

C. Purification of S 31794/F—1 Nucleus by Reversed-Phase Liquid Chromatography

Crude S 31794/F—1 nucleus, obtained as described in Section B, is purified according to the process of Section C of Example 4.

On the basis of absorption at 280 nm, fractions containing S 31794/F—1 nucleus are combined, evaporated under vacuum and lyophilized to give purified S 31794/F—1 nucleus.

Preparation of an Abbott and Fukuda Derivatives

The following procedure illustrates the preparation of the compounds of formula III by the "active ester" method. The specific compounds prepared by this procedure are the compounds of formula III wherein $R^5$ is $CH_3(CH_2)_{11}$—, and $R^1$, $R^2$, $R^3$ and $R^4$ are as in the respective nuclei.

Reference Preparation 1

Preparation of 2,4,5-Trichlorophenyl Tridecanoate

A solution of *n*-tridecanoic acid (Sigma Chemical Co, 12.5 g), 2,4,5-trichlorophenol (11.5 g), and N,N'-dicyclohexylcarbodiimide (12.0 g) in methylene chloride (650 ml) is stirred at room temperature for 16 hours. The reaction mixture is then filtered and dried *in vacuo* to give 2,4,5-trichlorophenyl tridecanoate (22 g). The material is purified by column chromatography over silica gel (Woelm) using toluene as the eluent. Fractions are monitored by TLC using a shortwave UV light for detection. Fractions containing the purified product are pooled and concentrated *in vacuo* to dryness.

Reference Example 1

Acylation of A—30912A Nucleus with 2,4,5-Trichlorophenyl n-Tridecanoate

A solution of 2,4,5-trichlorophenyl tridecanoate (6.0 g) and A—30912A nucleus (4.5 g) in dimethylformamide (DMF) (600 ml) is stirred at room temperature for 16 hours. Removal of solvent *in vacuo* affords a residue (12 g). The residue is slurried with methylene chloride (500 ml) for 45 minutes, and the mixture is filtered. The filtrate is discarded. The remaining solids are extracted with methanol (500 ml). The methanol extract is filtered and concentrated *in vacuo* to give a crude product (5.0 g).

The crude product is purifed by reversed phase HPLC as follows:

A sample of the crude product (1 g), dissolved in methanol (5 ml), is injected into a 1- x 32-in stainless steel column packed with LP—1/$C_{18}$ resin (see Preparations 10 and 11). The column is eluted with a solvent system comprising $H_2O$:$CH_3OH$:$CH_3CN$ (3:3:4). The elution is performed at a pressure of 1000—1500 psi (69—103 bar) with a flow rate of 11—12 ml/min using an LDC duplex pump (Milton-Roy). The effluent is monitored by an ultraviolet detector (ISCO—UA—5) at 280 nm. Fractions are collected every two minutes (21—24 ml). The fractions containing the desired product are pooled and dried *in vacuo*. Yield of the product: 550 mg. The above-described chromatography is repeated four times to give additional purified samples of the product as follows: 620 mg, 520 mg, 670 mg, and 490 mg to give a total weight of 2.8 g.

Following the above procedure 40 g of A—30912A nucleus is reacted with 2,4,5-trichlorophenyl *n*-tridecanoate to give 2.6 g of purified title product. The materials from both preparations (5.4 g) are combined. Mass ion by FDMS ($M^+ + Na^+$): 1016. (Theoretical: $M^+ + Na^+ = 1016$). Analytical HPLC ($C_{18}$ Micro Bondapak, Waters Co.) with eluent system $H_2O$:$CH_3OH$:$CH_3CN$ (2:1:2) shows only one peak.

Reference Example 2

Acylation of A—30912B Nucleus with 2,4,5-Trichlorophenyl n-Tridecanoate

A solution of 2,4,5-trichlorophenyl *n*-tridecanoate (3.3 mmoles) and A—30912B nucleus (1 mmole) in dimethylformamide (DMF) (200 ml) is stirred at room temperature for 16 hours. Removal of solvent *in vacuo* affords a residue. The residue is slurried with methylene chloride (300 ml) for 45 minutes, and the mixture is filtered. The filtrate is discarded. The remaining solids are extracted with methanol (300 ml), and the methanol extract is filtered and concentrated *in vacuo* to give a crude product.

The crude product is purified by reversed-phase HPLC as follows:

A sample of the crude product (1 g), dissolved in methanol (5 ml), is injected into a 1- x 32-inch stainless steel column packed with LP—1/$C_{18}$ resin (see Preparations 10 and 11). The column is eluted with a solvent system comprising 3:3:4 $H_2O/CH_3OH/CH_3CN$. The elution is performed at a pressure of 1000—1500 psi with a flow rate of 11—12 ml/min using an LDC duplex pump (Milton-Roy). The effluent is monitored by a UV detector (ISCO—UA—5) at 280 nm. Fractions are collected every two minutes (21—24 ml). The fractions containing the desired product are pooled and dried *in vacuo* to afford the title product. The purified product is analyzed by TLC using reversed-phase $C_{18}$ plates (Whatman $KC_{18}$) and a solvent system comprising 1:2:2 (v/v) $H_2O/CH_3OH/CH_3CN$. After development, the plates are observed under UV light to detect the product.

Reference Example 3

Acylation of A—30912D Nucleus with 2,4,5-Trichlorophenyl n-Tridecanoate

A solution of 2,4,5-trichlorophenyl *n*-tridecanoate (3.3 mmoles) and A—30912D nucleus (1 mmole) in dimethylformamide (DMF) (200 ml) is stirred at room temperature for 16 hours. Removal of

solvent *in vacuo* affords a residue. The residue is slurried with methylene chloride (300 ml) for 45 minutes, and the mixture is filtered. The filtrate is discarded. The remaining solids are extracted with methanol (300 ml), and the methanol extract is filtered and concentrated *in vacuo* to give a crude product.

The crude product is purified by reversed-phase HPLC as described in Reference Example 2.

Reference Example 4

Acylation of A—30912H Nucleus with 2,4,5-Trichlorophenyl n-Tridecanoate

A solution of 2,4,5-trichlorophenyl *n*-tridecanoate (3.3 mmoles) and A—30912H nucleus (1 mmole) in dimethylformamide (DMF) (200 ml) is stirred at room temperature for 16 hours. Removal of solvent *in vacuo* affords a residue. The residue is slurried with methylene chloride (300 ml) for 45 minutes, and the mixture is filtered. The filtrate is discarded. The remaining solids are extracted with methanol (300 ml), and the methanol extract is filtered and concentrated *in vacuo* to give a crude product.

The crude product is purified by reversed-phase HPLC as described in Reference Example 2.

Reference Example 5

The compound of formula III wherein $R^3$ is *n*-propoxy and $R^5$ is $CH_3—(CH_2)_{11}—$, prepared according to the procedure of Reference Example 4, but using as a starting material the compound of formula I wherein $R^3$ is *n*-propoxy (prepared by the method of Example 12).

Reference Example 6

Acylation of S 31794/F—1 Nucleus with 2,4,5-Trichlorophenyl n-Tridecanoate

A solution of 2,4,5-trichlorophenyl *n*-tridecanoate (3.3 mmoles) and S 31794/F—1 nucleus (1 mmole) in dimethylformamide (DMF) (200 ml) is stirred at room temperature for 16 hours. Removal of solvent *in vacuo* affords a residue. The residue is slurried with methylene chloride (300 ml) for 45 minutes, and the mixture is filtered. The filtrate is discarded. The remaining solids are extracted with methanol (300 ml), and the methanol extract is filtered and concentrated *in vacuo* to give a crude product.

The crude product is purified by reversed-phase HPLC as described in Reference Example 2.

Preparation of Debono Group I Derivatives

The following procedure, which gives the preparation of the compounds of formula III wherein $R^5$ is

$CH_3(CH_2)_{10}CONH$ 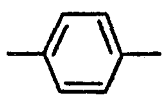

illustrates the method of preparation of the Debono I compounds of formula III.

Reference Preparation 2

Preparation of N-(*n*-Dodecanoyl)-*p*-aminobenzoic Acid

*n*-Dodecanoyl chloride (8.74 g; 40 mmoles) is added dropwise to a solution of *p*-aminobenzoic acid (5.5 g; 40 mmoles) dissolved in pyridine (100 ml). The mixture is stirred for 3 hours and poured into water (3 L). The precipitate which forms is filtered and dried *in vacuo* to give N-(*n*-dodecanoyl)-*p*-aminobenzoic acid (11.01 g).

Reference Preparation 3

Preparation of the 2,4,5-Trichlorophenyl Ester of N-(*n*-Dodecanoyl)-*p*-aminobenzoic Acid

N-(*n*-Dodecanoyl)-*p*-aminobenzoic acid (11.01 g; 34.5 mmoles), 2,4,5-trichlorophenol (7.5 g; 38 mmoles), and N,N'-dicyclohexylcarbodiimide (6.94 g; 34.5 mmoles) are dissolved in methylene chloride (250 ml). The mixture is stirred at room temperature for 3.5 hours and then filtered. The filtrate is evaporated *in vacuo* to give a residue which is crystallized from acetonitrile/water to afford the 2,4,5-trichlorophenyl ester of N-(*n*-dodecanoyl)-*p*-aminobenzoic acid (12.84 g).

Reference Example 7

Acylation of A—30912A Nucleus

A—30912A nucleus (8.16 g; 10.2 mmoles) and the 2,4,5-trichlorophenyl ester of N-(*n*-dodecanoyl)-*p*-aminobenzoic acid (4.72 g; 10.2 mmoles) are dissolved in dimethylformamide (100 ml). The solution is stirred at room temperature for 15 hours. Solvent is removed *in vacuo* to give a residue which is washed twice with diethyl ether. The washes are discarded. The washed residue is dissolved in methanol (50 ml) and is purified by reversed phase HPLC by means of a "Prep LC/System 500" unit (Waters Associates, Inc., Milford, Mass.) using a Prep Pak-500/$C_{18}$ column (Waters Associates, Inc.,) as the stationary phase. The column is eluted isocratically with $H_2O:CH_3OH:CH_3CN$ (25:65:10 v/v) at 500

psi. The fractions are analyzed by TLC using silica gel plates and $H_2O:CH_3OH:CH_3CN$ (25:65:10 v/v) as the solvent system. Fractions containing the desired product are combined and lyophilized to give the N-(*n*-dodecanoyl)-*p*-aminobenzoyl derivative of A-30912A nucleus (3.5 g).

Reference Example 8

Acylation of A—30912B Nucleus

A—30912B nucleus (10.2 mmoles) and the 2,4,5-trichlorophenyl ester of N-(*n*-dodecanoyl)-*p*-aminobenzoic acid (10.2 mmoles) are dissolved in dimethylformamide (100 ml). The solution is stirred at room temperature for 15 hours. Solvent is removed *in vacuo* to give a residue which is washed twice with diethyl ether. The washes are discarded. The washed residue is dissolved in methanol (50 ml) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(*n*-dodecanoyl)-*p*-aminobenzoyl derivative of A—30912B nucleus.

Reference Example 9

Acylation of A—30912D Nucleus

A—30912D nucleus (10.2 mmoles) and the 2,4,5-trichlorophenyl ester of N-(*n*-dodecanoyl)-*p*-aminobenzoic acid (10.2 mmoles) are dissolved in dimethylformamide (100 ml). The solution is stirred at room temperature for 15 hours. Solvent is removed *in vacuo* to give a residue which is washed twice with diethyl ether. The washes are discarded. The washed residue is dissolved in methanol (50 ml) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(*n*-dodecanoyl)-*p*-aminobenzoyl derivative of A—30912D nucleus.

Reference Example 10

Acylation of A—30912H Nucleus

A—30912H nucleus (10.2 mmoles) and the 2,4,5-trichlorophenyl ester of N-(*n*-dodecanoyl)-*p*-aminobenzoic acid (10.2 mmoles) are dissolved in dimethylformamide (100 ml). The solution is stirred at room temperature for 15 hours. Solvent is removed *in vacuo* to give a residue which is washed twice with diethyl ether. The washes are discarded. The washed residue is dissolved in methanol (50 ml) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi (35 bar). The fractions are analyzed by TLC using silica gel plates and $H_2O/CH_3OH/CH_3CN$ (25:65:10 v/v) as the solvent system. Fractions containing the desired product are combined and lyophilized to give the N-(*n*-dodecanoyl)-p-aminobenzoyl derivative of A—30912H nucleus.

Reference Example 11

The compound of formula III wherein $R^3$ is isobutoxy and $R^5$ is

$CH_3(CH_2)_{10}CONH$ 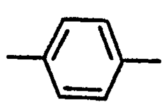

prepared according to the procedure of Reference Example 9, but using as a starting material the compound of formula I wherein $R^3$ is isobutoxy (prepared by the method of Example 13).

Reference Example 12

Acylation of S 31794/F—1 Nucleus

S 31794/F—1 nucleus (10.2 mmoles) and the 2,4,5-trichlorophenyl ester of N-(*n*-dodecanoyl)-*p*-aminobenzoic acid (10.2 mmoles) are dissolved in dimethylformamide (100 ml). The solution is stirred at room temperature for 15 hours. Solvent is removed *in vacuo* to give a residue which is washed twice with diethyl ether. The washes are discarded. The washed residue is dissolved in methanol (50 ml) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi (35 bar). The fractions are analyzed by TLC using silica gel plates and $H_2O/CH_3OH/CH_3CN$ (25:65:10 v/v) as the solvent system. Fractions containing the desired product are combined and lyophilized to give the N-(*n*-dodecanoyl)-*p*-aminobenzoyl derivative of S 31794/F—1 nucleus.

Preparation of a Debono Group II Derivative

The following procedure, which gives the preparation of the compounds of formula III wherein $R^5$ is

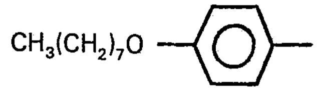

illustrates the process for the preparation of the Debono II compounds of formula III.

Reference Preparation 4

Preparation of *p*-(*n*-Octyloxy)benzoic Acid

A solution of *p*-hydroxybenzoic acid (19.2 g, 150 mmoles) in 10% aqueous sodium hydroxide (120 ml) is added to dimethyl sulfoxide (DMSO) (480 ml) previously heated to 80°C. *n*-Octyl bromide (28.95 g, 150 mmoles) is added dropwise to the solution. The reaction mixture is stirred for 4 hours at room temperature after which it is poured into ice water (1200 ml). Conc. hydrochloric acid (30 ml) is added, and the mixture is allowed to stand until precipitation is complete. The precipitate is collected, dried, and crystallized from acetonitrile-water. mp 97—99°C.

Analysis for $C_{15}H_{22}O_3$:

Calculated: C, 71.97; H, 8.86
Found: C, 71.72; H, 9.10

Reference Preparation 5

Preparation of the 2,4,5-Trichlorophenyl Ester of p-(n-Octyloxy)benzoic Acid

*p*-(*n*-Octyloxy)benzoic acid (6.18 g, 24.7 mmoles), 2,4,5-trichlorophenol (5.39 g, 27.2 mmoles), and N,N'-dicyclohexylcarbodiimide (4.94 g, 24.7 mmoles) are dissolved in methylene chloride (200 ml). The mixture is stirred at room temperature for 18 hours and then is filtered. The filtrate is evaporated to give an oil, which is crystallized from $CH_3CN$:$H_2O$ to give the 2,4,5-trichlorophenyl ester of *p*-(*n*-octyloxy)benzoic acid.

NMR Analysis: δ4.02 (2H, t, J = 3Hz), δ7.0 (1H, d, J = 4Hz), 7.23 (s, 1H), 7.3 (s, 1H), 8.08 (d, 1H, J = 4Hz).

Reference Example 13

Acylation of A—30912A Nucleus

A—30912A nucleus (14.2 g, 17.8 mmoles) and the 2,4,5-trichlorophenyl ester of *p*-(*n*-octyloxy)benzoic acid (15.32 g, 35.7 mmoles) are dissolved in dimethylformamide (150 ml). The solution is stirred at room temperature for 16—20 hours. Solvent is removed *in vacuo*, and the residue is washed twice with diethyl ether and twice with methylene chloride. The washes are discarded. The washed residue is dissolved in ethylacetate:methanol (1:3, 80 ml) and is purified by HPLC using a "Prep LC/System 500" unit with silica gel as the stationary phase. The column is eluted stepwise with methanol:ethyl acetate (1:4 to 2:3) solvent systems. The fractions are analyzed by silica gel (Merck) TLC using an ethyl acetate:methanol (3:2 v/v) as the solvent system. Fractions devoid of A—30912A nucleus are pooled and lyophilized to give the *p*-(*n*-octyloxy)benzoyl derivative of A—30912A nucleus. Yield: 7.13 g; $M^+$ +23: 1052 (by FDMS).

Reference Example 14

Acylation of A—30912B Nucleus

A—30912B nucleus (17.8 mmoles) and the 2,4,5-trichlorophenyl ester of *p*-(*n*-octyloxy)benzoic acid (35.7 mmoles) are dissolved in dimethylformamide (150 ml). The solution is stirred at room temperature for 16—20 hours. Solvent is removed *in vacuo*, and the residue is washed twice with diethyl ether and twice with methylene chloride. The washes are discarded. The washed residue is dissolved in ethyl acetate:methanol (1:3) (80 ml) and is purified by HPLC using a "Prep LC/System 500" unit, using silica gel as the stationary phase. The column is eluted stepwise with methanol:ethyl acetate (1:4 to 2:3) solvent systems. The fractions are analyzed by TLC using silica gel (Merck) and ethyl acetate:methanol (3:2 v/v) as the solvent system. Fractions devoid of A—30912B nucleus are pooled and lyophilized to give the *p*-(*n*-octyloxy)benzoyl derivative of A—30912B nucleus.

Reference Example 15

Acylation of A—30912D Nucleus

A—30912D nucleus (17.8 mmoles) and the 2,4,5-trichlorophenyl ester of *p*-(*n*-octyloxy)benzoic acid (35.7 mmoles) are dissolved in dimethylformamide (150 ml). The solution is stirred at room temperature for 16—20 hours. Solvent is removed *in vacuo*, and the residue is washed twice with diethyl ether and twice with methylene chloride. The washes are discarded. The washed residue is dissolved in ethyl acetate:methanol (1:3) (80 ml) and is purified by HPLC using a "Prep LC/System

500" unit, using silica gel as the stationary phase. The column is eluted stepwise with methanol:ethyl acetate (1:4 to 2:3) solvent systems. The fractions are analyzed by TLC using silica gel (Merck) and ethyl acetate:methanol (3:2 v/v) as the solvent system. Fractions devoid of A—30912B nucleus are pooled and lyophilized to give the *p*-(*n*-octyloxy)benzoyl derivative of A—30912D nucleus.

Reference Example 16

Acylation of A—30912H Nucleus

A—30912H nucleus (17.8 mmoles) and the 2,4,5-trichlorophenyl ester of *p*-(*n*-octyloxy)benzoic acid (35.7 mmoles) are dissolved in dimethylformamide (150 ml). The solution is stirred at room temperature for 16—20 hours. Solvent is removed *in vacuo*, and the residue is washed twice with diethyl ether and twice with methylene chloride. The washes are discarded. The washed residue is dissolved in ethyl acetate:methanol (1:3) (80 ml) and is purified by HPLC using a "Prep LC/System 500" unit, using silica gel as the stationary phase. The column is eluted stepwise with methanol:ethyl acetate (1:4 to 2:3) solvent systems. The fractions are analyzed by TLC using silica gel (Merck) and ethyl acetate:methanol (3:2 v/v) as the solvent system. Fraction devoid of A—30912H nucleus are pooled and lyophilized to give the *p*-(*n*-octyloxy)benzoyl derivative of A—30912H nucleus.

Reference Example 17

The compound of formula III wherein $R^3$ is *n*-hexyloxy and $R^5$ is

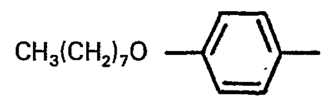

prepared according to the procedure of Reference Example 16, but using as a starting material the compound of formula I wherein $R^3$ is *n*-hexyloxy (prepared by the method of Example 15).

Reference Example 18

Acylation of S 31794/F—1 Nucleus

S 31794/F—1 nucleus (17.8 mmoles) and the 2,4,5-trichlorophenyl ester of *p*-(*n*-octyloxy)benzoic acid (35.7 mmoles) are dissolved in dimethylformamide (150 ml). The solution is stirred at room temperature for 16—20 hours. Solvent is removed *in vacuo*, and the residue is washed twice with diethyl ether and twice with methylene chloride. The washes are discarded. The washed residue is dissolved in ethyl acetate:methanol (1:3) (80 ml) and is purified by HPLC using a "Prep LC/System 500" unit, using silica gel as the stationary phase. The column is eluted stepwise with methanol:ethyl acetate (1:4 to 2:3) solvent systems. The fractions are analyzed by TLC using silica gel (Merck) and ethyl acetate:methanol (3:2 v/v) as the solvent system. Fractions devoid of S 31794/F—1 nucleus are pooled and lyophilized to give the *p*-(*n*-octyloxy)benzoyl derivative of S 31794/F—1 nucleus.

Reference Example 19

The antifungal activity of the compounds of formula III can be demonstrated *in vitro* in standard disc-diffusion tests and agar-dilution tests and *in vivo* in standard tests in mice which assess effectiveness against a systemic fungal infection. The results of the antifungal testing of representative compounds of formula III are set forth in Tables IV through VIII.

Tables IV and V give the results of the testing *in vitro* of the compounds of Reference Examples 1, 7 and 13 by agar-plate disc-diffusion methods. In Table IV activity is measured by the size (diameter in mm) of the observed zone of inhibition of the microorganism produced by the test compound. In Table V activity is measured by the minimal inhibitory concentration (MIC) of the substance (mg/disc) required to inhibit growth of the test organism. Table VI gives the results of the testing *in vitro* of the *p*-(*n*-octyloxy)benzoyl derivative of A—30912A nucleus against five strains of *Candida albicans* by the agar-dilution method. In Table VI activity is measured by the minimal inhibitory concentration (MIC) of the substance (mg/ml) required to inhibit the test organism.

The results of *in vivo* tests to evaluate the effectiveness of the derivatives against an infection caused by *Candida albicans* A—26 in mice are given in Table VII. In these tests activity is measured by the $ED_{50}$ value (the dose in mg/kg required to cure 50% of the test animals). Where an $ED_{50}$ value was not obtained, activity is indicated by the lowest dose at which a significant antifungal effect is observed. In these tests, groups of male albino mice (specific pathogen free), weighing 18 to 20 grams, are infected intravenously with *Candida albicans* A—26. The animals are X-irradiated 24 hours prior to infection at about 50 roentgens per minute for 8 minutes (400 total dose) to reduce immune responses to the infecting organism. At 0, 4, and 24 hours post infection each group of mice is given graded doses subcutaneously of the test compound as a suspension in 33% polyethylene glycol(PEG)-water. The day of death for each animal is recorded. Student's t test statistical comparison of the average day of death is made between each group of infected-treated animals at a particular dosage level and 10 infected-

untreated animals to determine if treatment significantly extends survival time.

Table VIII gives the results of the testing of the compounds of Reference Examples 1, 7 and 13 for absorption after oral administration. In these tests, mice are gavaged with a dose of 416 mg/kg of the test compound suspended in 33% PEG 400-water. At time intervals, blood samples are taken from the orbital sinus and are assayed for antibiotic activity as follows: A 7-mm disc containing 20 ml of whole blood is placed on agar seeded with *Aspergillus montevidensis* A35137. After 40 hours incubation at 30°C, zones of inhibition from the blood samples are compared to a standard obtained from the test compound, and the amount of compound in the blood sample is calculated.

TABLE IV

Derivatives of A—30912A Nucleus

Antifungal Activity by the Agar-Plate Disc-Diffusion Test

| Reference Example No. | Compounds of Formula III<br>$R^5$ = | Size of Zone of Inhibition (mm)[a]<br>Saccharomyces pastorianus X—52 | Neurospora crassa 846 | Trichophyton mentagrophytes A—23 | Candida albicans A—26 |
|---|---|---|---|---|---|
| 1 | $CH_3(CH_2)_{11}$— | 21 | 32 | 60* | 30 |
| 7 | $CH_3(CH_2)_{10}CONH$ 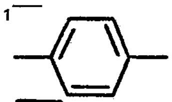 | 21<br>17 | 33*<br>35* | 55*<br>56* | 23<br>19 |
| 13 | $CH_3)CH_2)_7O$ 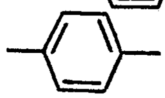 | 18 | 23* | — | 28 |

*Distinct measurable zone of inhibition with regrowth of organism around disc.

[a]Compounds were tested as methanol suspensions at a concentration of 1 mg/ml by dipping a 7-mm disc into the suspension and placing it on the agar surface.
Incubation: 24—48 hours at 25—37°C.

TABLE V

Derivatives of A—30912A Nucleus

Antifungal Activity by the Agar-plate Disc-Diffusion Test

| Reference Example No. | Compounds of Formula III<br>$R^5$ = | MIC (mg/disc)*<br>Candida albicans A—26 | Trichophyton mentagrophytes #6 |
|---|---|---|---|
| 1 | $CH_3(CH_2)_{11}$— | 0.625 | 0.039 |
| 7 | $CH_3(CH_2)_{10}CONH$ 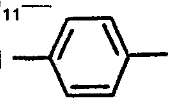 | 0.625<br>1.25 | >0.039<br>0.678 |
| 13 | $CH_3(CH_2)_7O$ 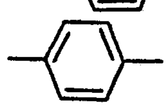 | 0.156<br>>0.302(0.312)** | |

*Compounds were suspended in 0.01M sodium borate solution, pH 7.5. The compounds were tested at 20 mg/disc at top level and at two-fold dilutions until end points were reached. Incubation: 24 hours; 30°C.

**Against 5 isolates

TABLE VI

*In vitro* Activity of Derivate of A—30912A Nucleus wherein $R^5$=$CH_3(CH_2)_7O$ 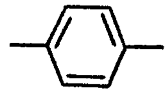 against *Candida albicaus* Strains.

| MIC (μg/ml) | | | | |
|---|---|---|---|---|
| A26 | SBH 16 | SBH 31 | SBH 28 | SBH 29 |
| 0.312 | 0.312 | 0.312 | 0.312 | 0.312 |

TABLE VII

Derivatives of A—30912A Nucleus

Therapeutic Activity Against *C. Albicans* in Mice

| Reference Example No. | Compounds of Formula III $R^5$= | Dosage Schedule* | $ED_{50}$ (mg/kg) | Lowest Active Dose (mg/kg) |
|---|---|---|---|---|
| 1 | $CH_3(CH_2)_{11}$— | B | 34 | 20 |
| 7 | $CH_3(CH_2)_{10}CONH$ 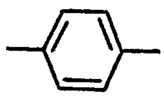 | A | 15 | 10 |
| | | | 15 | ≥5 |
| 13 | $CH_3(CH_2)_7O$ 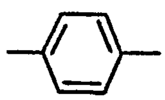 | A | 13 | 10 |
| | | | 22.2 | >12.5 |

*Dosage Schedules: A=40, 20, 15, and 10 mg/kg; B=80, 40, 20 and 10 mg/kg. Dosage given 0,4, and 24 hours post injection as suspension of test compound in 30% PEG-$H_2O$. Number of mice receiving test compounds at each dosage level: 6 mice per group. Number of mice in control (untreated) group: 10 mice per group.

**As measured by increase in survival time of treated animals versus control, calculated by method of L. J. Reed and H. Muench, *Amer. J. Hygiene, 27,* 493—497 (1938).

TABLE VIII

Derivatives of A-30912A Nucleus

Blood Levels After Oral Administration in Mice

| Reference Example No. | Compounds of Formula III $R^5$= | Blood Levels* (mg/ml) |
|---|---|---|
| 1 | $CH_3(CH_2)_{11}$— | 0.10 |
| 7 | $CH_3(CH_2)_{10}CONH$ 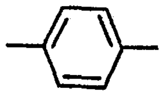 | 0.83 |
| 13 | $CH_3(CH_2)_7O$ 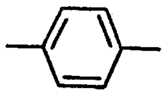 | 23. |

*Four hours after administration of test compound at dose of 416 mg/kg by gavage as suspension of compound in 33% PEG 400—$H_2O$. Compound determined by bioassay vs. *Aspergillus montevidensis* A-35137.

**Claims**

1. A cyclic peptide nucleus of the formula

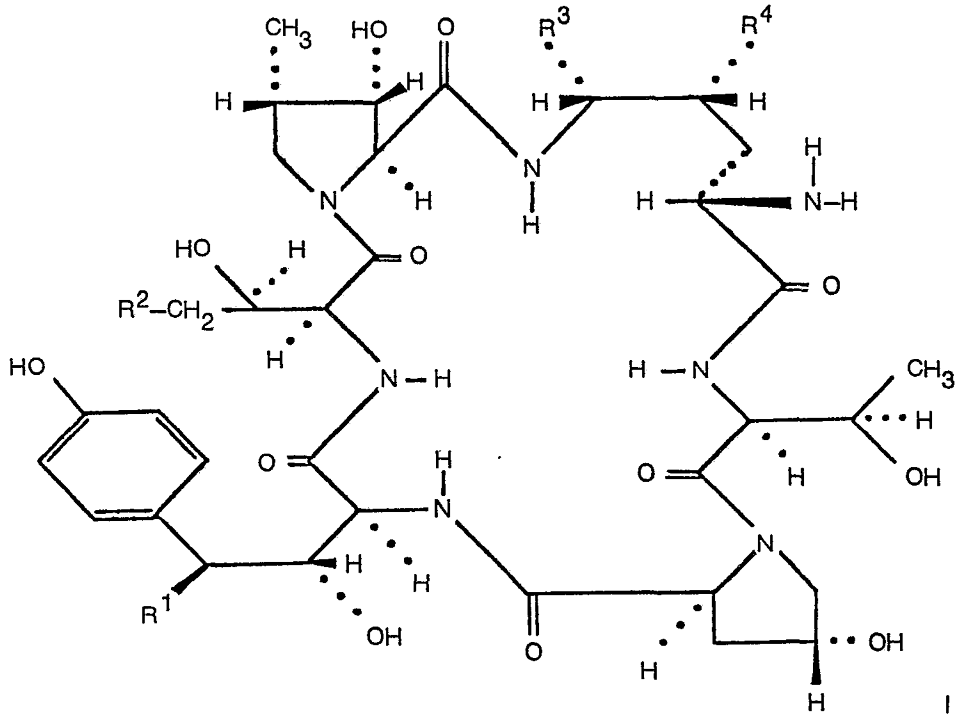

I

wherein

$R^1$ is H or OH and;

when $R^1$ is H, $R^2$ is H and $R^3$ and $R^4$ are both H or both OH, and

when $R^1$ is OH, $R^2$ is H, $R^3$ is OH or $C_1$—$C_6$ alkyloxy and $R^4$ is OH, or $R^2$ is

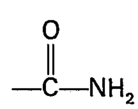

and $R^3$ and $R^4$ are both OH, and the acid addition salts thereof.

2. The nucleus A-30912A of claim 1 wherein in Formula I, $R^1$, $R^3$ and $R^4$ are all OH and $R^2$ is H.

3. The nucleus A-30912B of claim 1 wherein in formula I, $R^1$ and $R^2$ are both H and $R^3$ and $R^4$ are both OH.

4. The nucleus A-30912D of claim 1 wherein in Formula I, $R^1$, $R^2$, $R^3$ and $R^4$ are all H.

5. The nucleus A-30912H of claim 1 wherein in Formula I, $R^1$ and $R^4$ are both OH, $R^2$ is H and $R^3$ is $CH_3O$—.

6. The nucleus A-30912H-type of claim 1 wherein in Formula I, $R^1$ and $R^4$ are both OH, $R^2$ is H and $R^3$ is $C_2$—$C_6$ alkyloxy.

7. The nucleus A-30912H-type of claim 6 wherein $R^3$ is ethoxy, *n*-propoxy, isopropoxy, *n*-butoxy, isobutoxy, *tert*-butoxy, *n*-pentyloxy, *n*-hexyloxy, 2-ethyl-1-butoxy or 3-methyl-1-butoxy.

8. The nucleus S 31794/F—1 of claim 1 wherein in Formula I, $R^1$, $R^3$ and $R^4$ are all OH and $R^3$ is

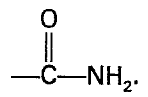

9. The acid addition salts of the nuclei of any one of claims 2 to 8.

10. A process for the preparation of a cyclic peptide nucleus of Formula I as defined in any one of claims 1—9 and the acid addition salts thereof, which comprises contacting in an aqueous medium a cyclic peptide antibiotic of Formula

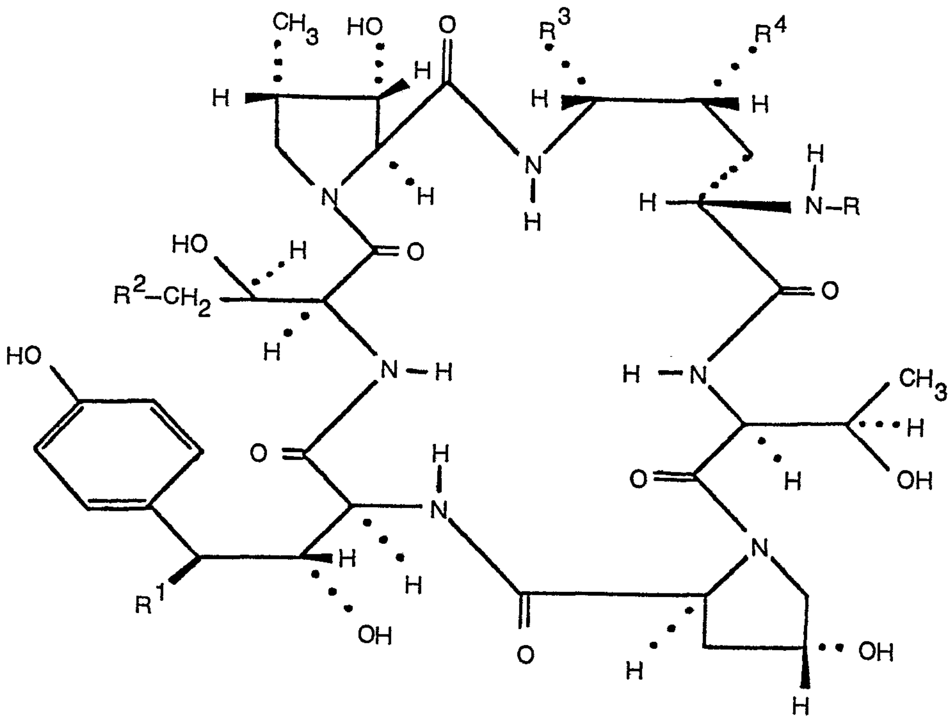

II

wherein $R^1$ is H or OH and,

when $R^1$ is H, $R^2$ is H and $R^3$ and $R^4$ are both H or both OH and R is stearoyl or linoleoyl, and

when $R^1$ is OH, $R^2$ is H and $R^4$ is OH, $R^3$ is OH and R is stearoyl, linoleoyl or palmitoyl, or $R^3$ is $C_1$—$C_6$ alkyloxy and R is stearoyl or linoleoyl, and

when $R^1$, $R^3$, $R^4$ are OH and $R^2$ is

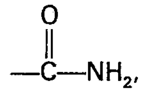

R is myristoyl,

with a deacylating enzyme produced by a microorganism of the family *Antinoplanaceae.*

11. The process of claim 10 wherein the microorganism of the family *Actinoplanaceae* is a member of the genus *Actinoplanes.*

12. The process of any one of claims 10 or 11 wherein the microorganism is *Actinoplanes utahensis.*

13. The process of claim 12 wherein the microorganism is *A. utahensis* NRRL 12052 or a mutant thereof which produces the enzyme.

14. The process of claim 13 wherein the microorganism is *A. utahensis* NRRL 12052.

15. The process of claim 10 wherein the microorganism is *Streptosporangium roseum* var. *hollandensis* NRRL 12064, or a mutant thereof which produces the enzyme.

16. The process of claim 15 wherein the microorganism is *Streptosporangium roseum* var. *hollandensis* NRRL 12064.

17. The process of claim 11 wherein the microorganism is *Actinoplanes missouriensis* NRRL 12053 or a mutant thereof which produces the enzyme.

18. The process of claim 17 wherein the microorganism is *Actinoplanes missouriensis* NRRL 12053.

19. The process of claim 11 wherein the microorganism is *Actinoplanes sp.* NRRL 12065 or a mutant thereof which produces the enzyme.

20. The process of claim 19 wherein the microorganism is *Actinoplanes sp.* NRRL 12065.

21. The process of claim 11 wherein the microorganism is *Actinoplanes sp.* NRRL 8122 or a mutant thereof which produces the enzyme.

22. The process of claim 21 wherein the microorganism is *Actinoplanes sp.* NRRL 8122.

23. A process of any one of claims 10 to 22 wherein the enzyme is present in a culture of the producing *Actinoplanaceae* microorganism.

24. The process of any one of claims 10 to 23 which comprises the additional step of isolating the cyclic peptide nucleus, or a salt thereof, from the fermentation mixture.

25. The process of any one of claims 10 to 24 which comprises the additional step of purifying the cyclic peptide nucleus, or a salt thereof.

**Revendications**

1. Noyau de peptide cyclique de formule:

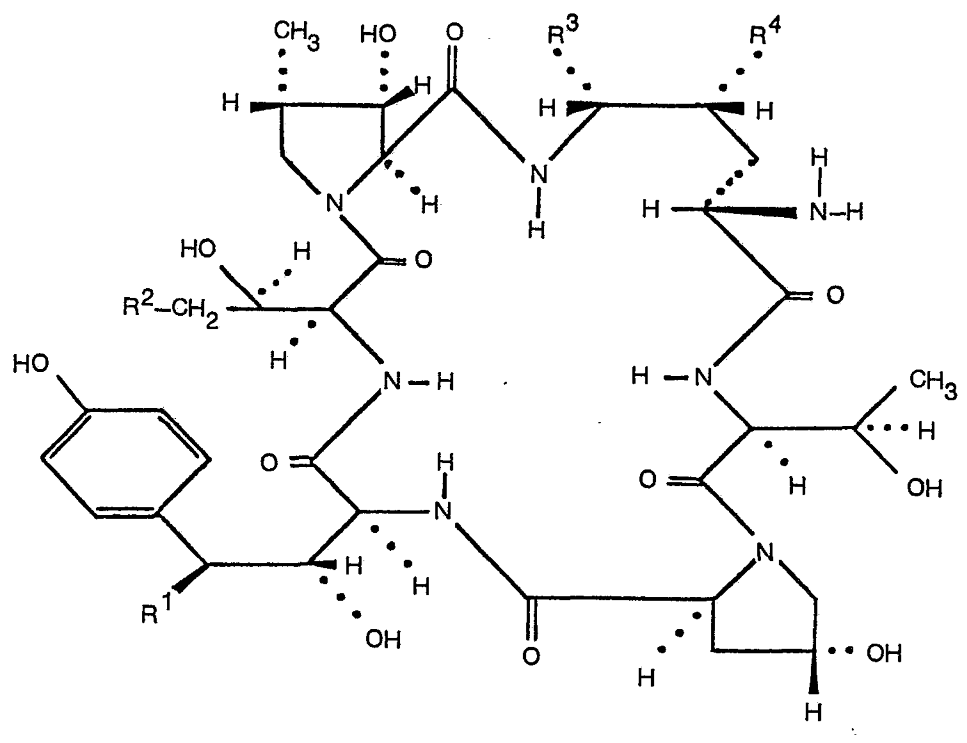

I

dans laquelle

$R^1$ représente H ou OH et

lorsque $R^1$ représente H, $R^2$ représente H, tandis que

$R^3$ et $R^4$ représentent tous deux H ou tous deux OH, et

lorsque $R^1$ représente OH, $R^2$ représente H, $R^3$ représente OH ou un groupe alkyloxy en $C_1$—$C_6$ et $R^4$ représente OH, ou $R^2$ représente

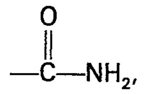

tandis que $R^3$ et $R^4$ représentent tous deux OH, de même que ses sels d'addition d'acide.

2. Noyau A-30912A suivant la revendication 1, caractérisé en ce que, dans la formule I, $R^1$, $R^3$ et $R^4$ représentent tous OH et $R^2$ représente H.

3. Noyau A-30912B suivant la revendication 1, caractérisé en ce que, dans la formule I, $R^1$ et $R^2$ représentent tous deux H, tandis que $R^3$ et $R^4$ représentent tous deux OH.

4. Noyau A-30912D suivant la revendication 1, caractérisé en ce que, dans la formule I, $R^1$, $R^2$, $R^3$ et $R^4$ représentent tous H.

5. Noyau A-30912H suivant la revendication 1, caractérisé en ce que, dans la formule I, $R^1$ et $R^4$ représentent tous deux OH, $R^2$ représente H et $R^3$ représente $CH_3O$—.

6. Noyau de type A-30912H suivant la revendication 1, caractérisé en ce que, dans la formule I, $R^1$ et $R^4$ représentent tous deux OH, $R^2$ représente H et $R^3$ représente un groupe alkyloxy en $C_2$—$C_6$.

7. Noyau de type A-30912H suivant la revendication 6, caractérisé en ce que $R^3$ représente un groupe éthoxy, un groupe n-propoxy, un groupe isopropoxy, un groupe n-butoxy, un groupe isobutoxy, un groupe tert-butoxy, un groupe n-pentyloxy, un groupe n-hexyloxy, un group 2-éthyl-1-butoxy ou un groupe 3-méthyle-1-butoxy.

8. Noyau S 31794/F—1 suivant la revendication 1, caractérisé en ce que, dans la formule I, $R^1$, $R^3$ et $R^4$ représentent tous OH et $R^3$ représente

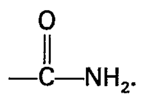

9. Sels d'addition d'acide des noyaux suivant l'une quelconque des revendications 2 à 8.

10. Procédé de préparation d'un noyau de peptide cyclique de formule I suivant l'une quelconque des revendications 1 à 9, ainsi que de ses sels d'addition d'acide, caractérisé en ce qu'il consiste à mettre un antibiotique de peptide cyclique de formule:

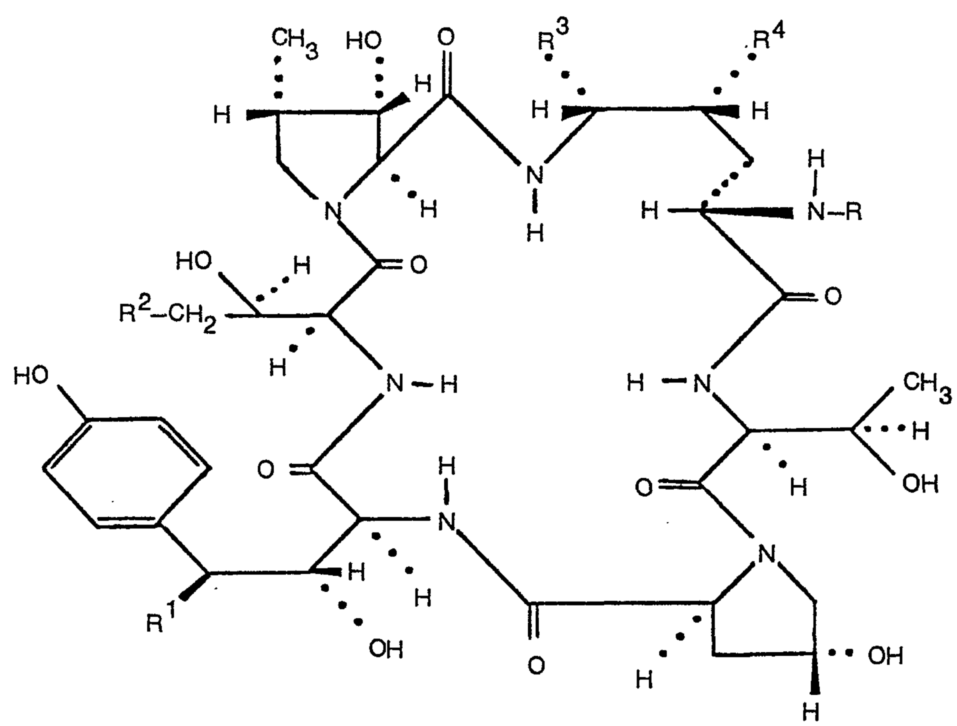

II

dans laquelle $R^1$ représente H ou OH et

lorsque $R^1$ représente H, $R^2$ représente H, tandis que $R^3$ et $R^4$ représentent tous deux H ou tous deux OH et R représente un groupe stéaroyle ou linoléoyle, et

lorsque $R^1$ représente OH, $R^2$ représente H et $R^4$ représente OH, $R^3$ représente OH et R représente

un groupe stéaroyle, linoléoyle ou palmitoyle, ou $R^3$ représente un groupe alkyloxy en $C_1$—$C_6$ et R représente un groupe stéaroyle ou linoléoyle, et

lorsque $R^1$, $R^3$ et $R^4$ représentent OH et que $R^2$ représente

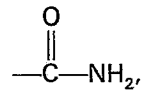,

R représente un groupe myristoyle, en contact, en milieu aqueux, avec une enzyme de désacylation produite par un micro-organisme de la famille *Actinoplanaceae.*

11. Procédé suivant la revendication 10, caractérisé en ce que le micro-organisme de la famille *Actinoplanaceae* est un membre du genre *Actinoplanes.*

12. Procédé suivant l'une quelconque des revendications 10 ou 11, caractérisé en ce que le micro-organisme est *Actinoplanes utahensis.*

13. Procédé suivant la revendication 12, caractérisé en ce que le micro-organisme est *A. utahensis* NRRL 12052 ou un de ses mutants produisant l'enzyme.

14. Procédé suivant la revendication 13, caractérisé en ce que le micro-organisme est *A. utahensis* NRRL 12052.

15. Procédé suivant la revendication 10, caractérisé en ce que le micro-organisme est *Streptosporangium roseum* var. *hollandensis* NRRL 12064 ou un de ses mutants produisant l'enzyme.

16. Procédé suivant la revendication 15, caractérisé en ce que le micro-organisme est *Streptosporangium roseum* var. *hollandensis* NRRL 12064.

17. Procédé suivant la revendication 11, caractérisé en ce que le micro-organisme est *Actinoplanes missouriensis* NRRL 12053 ou un de ses mutants produisant l'enzyme.

18. Procédé suivant la revendication 17, caractérisé en ce que le micro-organisme est *Actinoplanes missouriensis* NRRL 12053.

19. Procédé suivant la revendication 17, caractérisé en ce que le micro-organisme est *Actinoplanes sp.* NRRL 12065 ou un de ses mutants produisant l'enzyme.

20. Procédé suivant la revendication 19, caractérisé en ce que le micro-organisme est *Actinoplanes sp.* NRRL 12065.

21. Procédé suivant la revendication 11, caractérisé en ce que le micro-organisme est *Actinoplanes sp.* NRRL 8122 ou un de ses mutants produisant l'enzyme.

22. Procédé suivant la revendication 21, caractérisé en ce que le micro-organisme est *Actinoplanes sp.* NRRL 8122.

23. Procédé suivant l'une quelconque des revendications 10 à 22, caractérisé en ce que l'enzyme est présente dans une culture du micro-organisme producteur de la famille *Actinoplanaceae.*

24. Procédé suivant l'une quelconque des revendications 10 à 23, caractérisé en ce qu'il comprend l'étape supplémentaire consistant à isoler le noyau de peptide cyclique ou un de ses sels, du mélange de fermentation.

25. Procédé suivant l'une quelconque des revendications 10 à 24, caractérisé en ce qu'il comprend l'étape supplémentaire consistant à purifier le noyau de peptide cyclique ou un de ses sels.

**Patentansprüche**

1. Cyclischer Peptidkern der Formel I

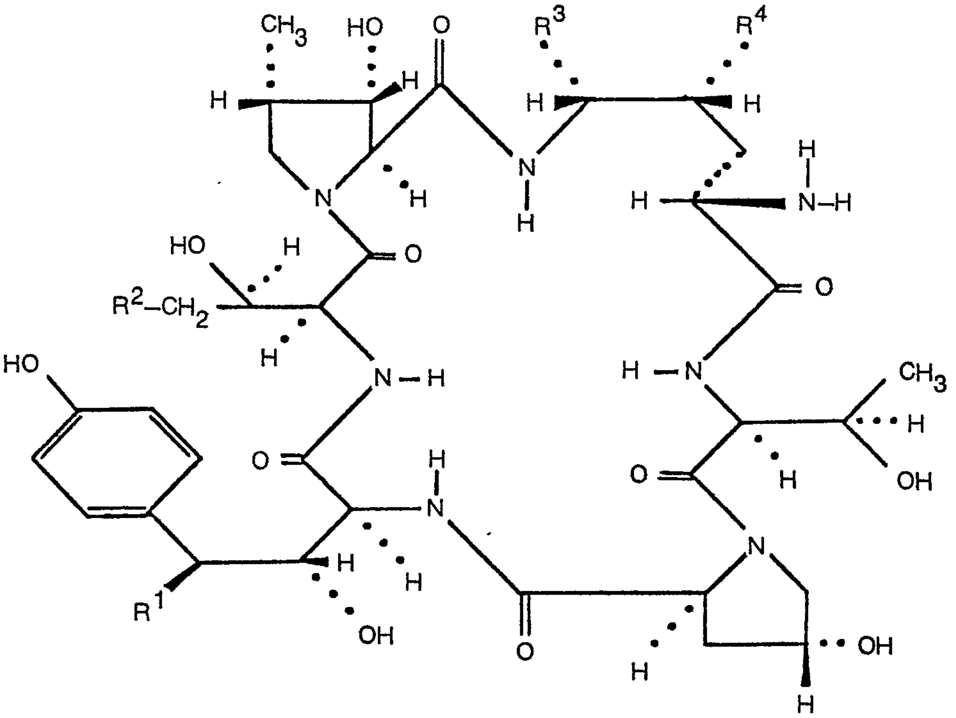

I

worin $R^1$ für H oder OH steht und, falls $R^1$ für H steht, $R^2$ dann H bedeutet und $R^3$ sowie $R^4$ jeweils H oder jeweils OH sind, oder falls $R^1$ für OH steht, $R^2$ dann H bedeutet, $R^3$ für OH oder $C_1$—$C_6$-Alkyloxy steht und $R^4$ für OH steht oder $R^2$ für

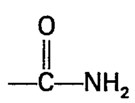

steht und $R^3$ sowie $R^4$ jeweils OH bedeuten, und die Säureadditionssalze hiervon.

2. Kern A-30912A gemäß Anspruch 1, dadurch gekennzeichnet, daß in der Formel I die Substituenten $R^1$, $R^3$ und $R^4$ alle OH sind und $R^2$ für H steht.

3. Kern A-30912B gemäß Anspruch 1, dadurch gekennzeichnet, daß in der Formel I die Substituenten $R^1$ und $R^2$ beide H sind und $R^3$ sowie $R^4$ beide für OH stehen.

4. Kern A-30912D gemäß Anspruch 1, dadurch gekennzeichnet, daß in der Formel I die Substituenten $R^1$, $R^2$, $R^3$ und $R^4$ alle für H stehen.

5. Kern A-30912H gemäß Anspruch 1, dadurch gekennzeichnet, daß in der Formel I die Substituenten $R^1$ und $R^4$ jeweils OH sind, $R^2$ für H steht und $R^3$ für $CH_3O$— steht.

6. Kern A-30912H gemäß Anspruch 1, dadurch gekennzeichnet, daß in der Formel I die Substituenten $R^1$ und $R^4$ jeweils OH sind, $R^2$ für H steht und $R^3$ für $C_2$—$C_6$-Alkyloxy steht.

7. Kern A-30912H gemäß Anspruch 6, dadurch gekennzeichnet, daß der Substituent $R^3$ für Ethoxy, n-Propoxy, Isopropoxy, n-Butoxy, Isobutoxy, tert-Butoxy, n-Pentyloxy, n-Hexyloxy, 2-Ethyl-1-butoxy oder 3-Methyl-1-butoxy steht.

8. Kern S 31794/F—1 gemäß Anspruch 1, dadurch gekennzeichnet, daß in der Formel I die Substituenten $R^1$, $R^3$ und $R^4$ alle OH sind und $R^3$ für

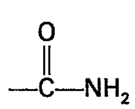

steht.

9. Säureadditonssalze der Kerne nach einem der Ansprüche 2 bis 8.

10. Verfahren zur Herstellung eines cyclischen Peptidkerns der Formel I gemäß einem der Ansprüche 1 bis 9 oder eines Säureadditionssalzes hiervon, dadurch gekennzeichnet, daß man in einem wässrigen Medium ein cyclisches Peptidantibiotikum der Formel II

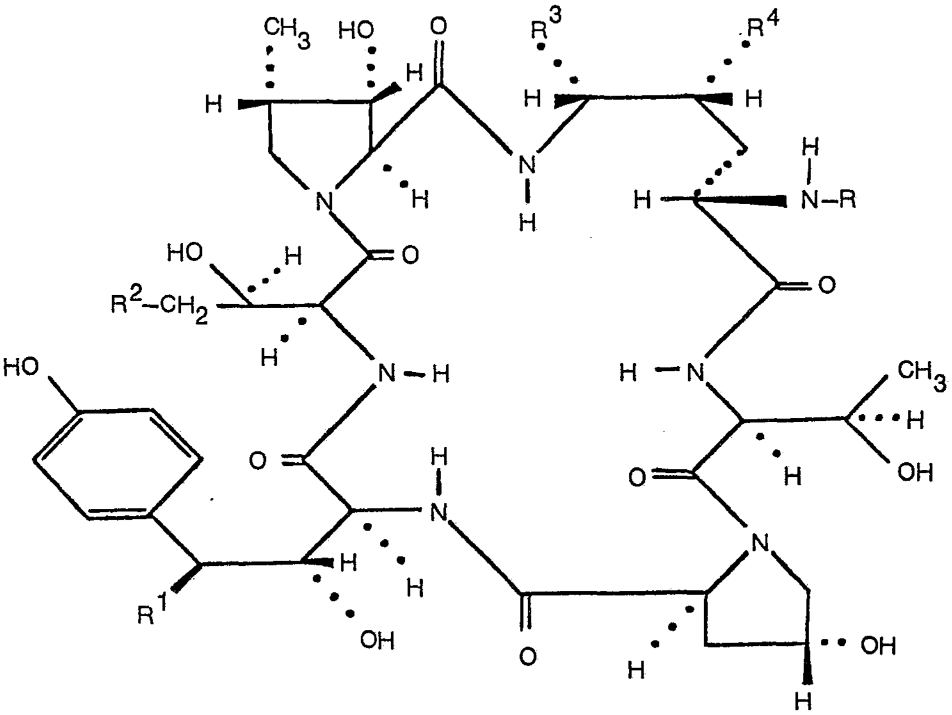

II

worin $R^1$ für H oder OH steht und, falls $R^1$ für H steht, $R^2$ dann H bedeutet und $R^3$ sowie $R^4$ jeweils H oder jeweils OH sind und R Stearoyl oder Linoleoyl bedeutet, oder, falls $R^1$ für OH steht, $R^2$ dann H ist, $R^4$ für OH steht, $R^3$ für OH steht und R Stearoyl, Linoleoyl oder Palmitoyl bedeutet oder $R^3$ für $C_1$—$C_6$-Alkyloxy steht und R Stearoyl oder Linoleoyl ist oder, falls $R^1$, $R^3$ und $R^4$ jeweils OH sind und $R^2$ für

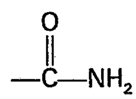

steht, R dann Myristoyl bedeutet, mit einem deacylierenden Enzym umsetzt, das von einem Mikroorganismus aus der Familie der Actinoplanaceten gebildet worden ist.

11. Verfahren nach Anspruch 10, dadurch gekennzeichnet, daß man als Mikroorganismus aus der Familie der Actinoplanaceten einen Vertreter des Genus Actinoplanes verwendet.

12. Verfahren nach einem der Ansprüche 10 oder 11, dadurch gekennzeichnet, daß man als Mikroorganismus Actinoplanes utahensis verwendet.

13. Verfahren nach Anspruch 12, dadurch gekennzeichnet, daß man als Mikroorganismus Actinoplanes utahensis NRRL 12052 oder eine das Enzym bildende Mutante hiervon verwendet.

14. Verfahren nach Anspruch 13, dadurch gekennzeichnet, daß man als Mikroorganismus Actinoplanes utahensis NRRL 12052 verwendet.

15. Verfahren nach Anspruch 10, dadurch gekennzeichnet, daß man als Mikroorganismus Streptosporangium roseum var. hollandensis NRRL 12064 oder eine das Enzym bildende Mutante hiervon verwendet.

16. Verfahren nach Anspruch 15, dadurch gekennzeichnet, daß man als Mikroorganismus Streptosporangium roseum var. hollandensis NRRL 12064 verwendet.

17. Verfahren nach Anspruch 11, dadurch gekennzeichnet, daß man als Mikroorganismus Actinoplanes missouriensis NRRL 12053 oder eine das Enzym bildende Mutante hiervon verwendet.

18. Verfahren nach Anspruch 17, dadurch gekennzeichnet, daß man als Mikroorganismus Actinoplanes missourienses NRRL 12053 verwendet.

19. Verfahren nach Anspruch 11, dadurch gekennzeichnet, daß man als Mikroorganismus Actinoplanes sp. NRRL 12065 oder eine das Enzym bildende Mutante hiervon verwendet.

20. Verfahren nach Anspruch 19, dadurch gekennzeichnet, daß man als Mikroorganismus Actinoplanes sp. NRRL 12065 verwendet.

21. Verfahren nach Anspruch 11, dadurch gekennzeichnet, daß man als Mikroorganismus Actinoplanes sp. NRRL 8122 oder eine das Enzym bildende Mutante hiervon verwendet.

22. Verfahren nach Anspruch 21, dadurch gekennzeichnet, daß man als Mikroorganismus Actinoplanes sp. NRRL 8122 verwendet.

23. Verfahren nach einem der Ansprüche 10 bis 22, dadurch gekennzeichnet, daß das Enzym in einer Kultur vorhanden ist, die Mikroorganismen aus der Familie der Actinoplanaceten bildet.

24. Verfahren nach einem der Ansprüche 10 bis 23, dadurch gekennzeichnet, daß man in einer weiteren Stufe aus dem Fermentationsgemisch den jeweiligen cyclischen Peptidkern oder das jeweilige Säureadditionssalz hiervon isoliert.

25. Verfahren nach einem der Ansprüche 10 bis 24, dadurch gekennzeichnet, daß man in einer weiteren Stufe den jeweiligen cyclischen Peptidkern oder das jeweilige Säureadditionssalz hiervon reinigt.

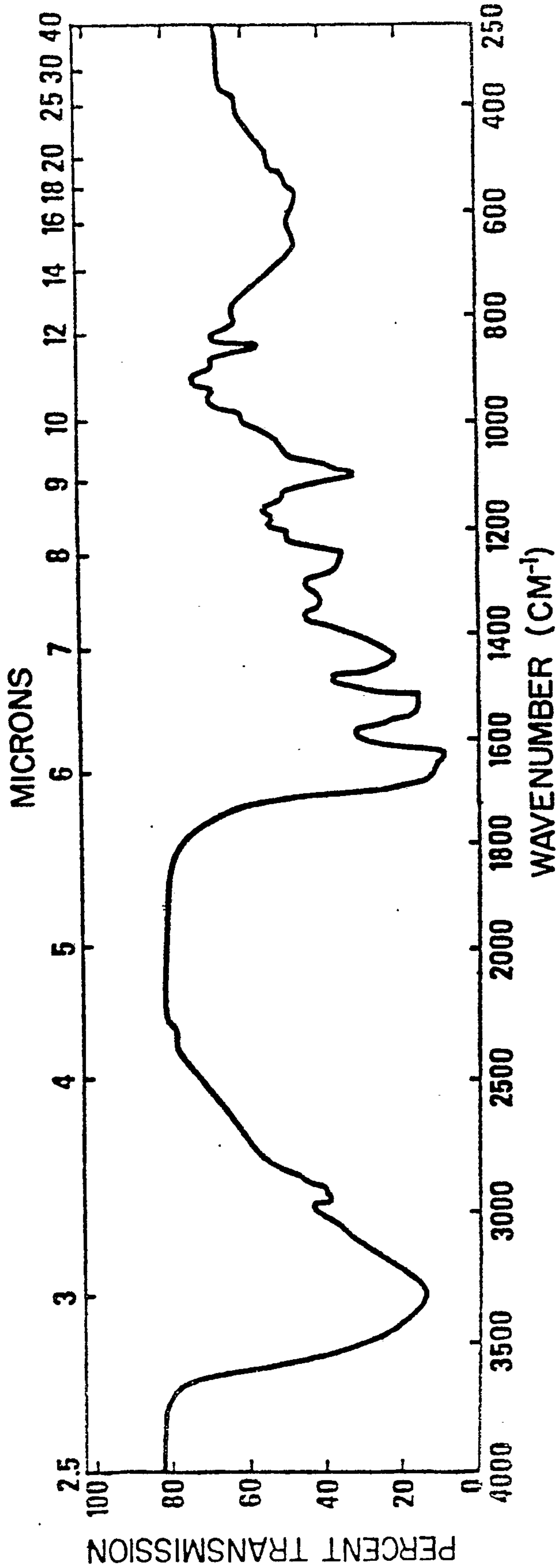
MICRONS
2.5 3 4 5 6 7 8 9 10 12 14 16 18 20 25 30 40
WAVENUMBER (CM-1)
4000 3500 3000 2500 2000 1800 1600 1400 1200 1000 800 600 400 250
PERCENT TRANSMISSION
100 80 60 40 20 0